(19) Europäisches Patentamt
European Patent Office
Office européen des brevets

(11) **EP 2 940 080 A2**

(12) **EUROPEAN PATENT APPLICATION**

(43) Date of publication:
**04.11.2015 Bulletin 2015/45**

(21) Application number: **15001320.9**

(22) Date of filing: **04.05.2015**

(51) Int Cl.:
**C08L 101/00** (2006.01)    **C08L 79/02** (2006.01)
**C08L 23/06** (2006.01)    **C09D 5/14** (2006.01)
**C09D 201/00** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **02.05.2014 GR 20140100263**

(71) Applicants:
• **NCSR DEMOKRITOS**
153 10 Athens (GR)
• **Papageorgiou, Sergios**
112 57 Kipseli Athens (GR)
• **Sideratou, Oreozili**
153 44 Gerakas Attikis (GR)
• **Katsaros, Fotios**
153 44 Gerakas Attikis (GR)

• **Stamatakis, Konstantinos**
153 43 Aghia Paraskevi Attikis (GR)

(72) Inventors:
• **Sidertou, Oreozili**
153 44 Gerakas Attikis (GR)
• **Katsaros, Fotios**
153 44 Gerakas Attikis (GR)
• **Stamatakis, Konstantinos**
153 43 Aghia Paraskevi (GR)
• **Papageorgiou, Sergios**
112 57 Kipseli Athens (GR)

(74) Representative: **Malamis, Alkisti-Irene**
**Malamis & Associates**
**Attorneys at Law**
**8 Palaia Tatoiou Street**
**146 71 Kifissia (GR)**

(54) **NOVEL DENDRITIC POLYMER- FUNCTIONALIZED NANOSTRUCTURED CARBON-BASED MATERIALS WITH ANTIBACTERIAL PROPERTIES AND THEIR EFFECT IN PHOTOSYNTHETIC PROCESS**

(57) The current invention presents the development of innovative antibacterial hybrid nanocomposites obtained by the interaction of nanostructured carbon-based materials with functionalized dendritic polymers through electrostatic interactions or other intermolecular forces. Their dispersions in various solvents exhibit extreme stability, even at high concentrations (up to 10 mg/mL), for long periods (more than one year) without precipitation. Additionally, in the present invention, the antibacterial properties of these novel hybrid nanocomposites as well as their effect on photosynthesis are described for the first time. Specifically, these nanocomposites exhibit enhanced antibacterial properties at very low concentration (5 $\mu$g/mL), while they cause an almost complete inhibition of Photosystem I in photosynthetic organisms. Furthermore, in this invention several applications of these antibacterial nanocomposites are presented and evaluated. Specifically, their incorporation or coating onto various materials and/or substrates, including polymers, metal, wood, woven and nonwoven textile materials, plastics, thermoplastics, synthetic or composite materials, etc., results in innovative surfaces with enhanced antibacterial properties. Finally, in order to evaluate and assess the antibacterial properties of the abovementioned nanocomposites and/or the surfaces resulting from their application, an *in-situ,* direct and instrumental method is invented, capable to quantify the antibacterial behaviour of any material, substrate or matrix at a relevant environment.

Printed by Jouve, 75001 PARIS (FR)

## Description

### Technical Field

The present invention deals with the development of functionalized nanostructured carbon-based materials with antibacterial properties as well as their effect on the photosynthetic process. Additionally, these hybrid materials can be incorporated or coated to any material or substrate (polymers, metal, wood, woven and nonwoven textile materials, plastics, thermoplastics, synthetic or composite materials, etc.) affording surfaces with antibacterial properties. Furthermore, an in situ, direct and instrumental method for the evaluation and assessment of antibacterial properties of types of materials or surfaces is described.

### Prior Art

In the recent years, antibacterial properties of carbon based nanomaterials have received special attention by the research community [i]. Previous studies on Single-Wall Carbon Nanotubes (hereinafter referred to as SWCTs) or Multi-Wall Carbon Nanotubes (hereinafter referred to as MWCTs) have revealed their significant antibacterial activity both on gram+ (gram positive) and gram- (gram negative) bacteria [ii,iii]. This antibacterial behavior was attributed to damages of the cell membrane caused by the interaction of the cells with Carbon Nanotubes (hereinafter referred to as CNTs) [iv]. The nano-sheets of Graphite oxide (GO) and graphene were found to inhibit the growth of the bacterium Escherichia coli (E. coli), while they exhibit reduced antibacterial activity against A549 cells [v], Furthermore, Arias and Yang found that while Double-Wall Carbon Nanotubes (hereinafter referred to as DWCNTs) do not exhibit antibacterial activity toward Gram positive and negative bacterial cells, SWCTs affect both types of bacteria [vi]. In another recent study, it was found that DWCNTs demonstrate antibacterial properties only together with surfactants, such as cetyltrimethylammonium bromide (hereinafter referred to as CTAB) or sodium dodecyl sulfate (hereinafter referred to as SDS) [vii]. In general, the antibacterial activity of CNTs can be improved, either by chemical modification, or by attaching metal nanoparticles (NPs) on their surface [vii,ix,x,xi]. For example CNTs modified with poly-lysine and other biomolecules, show antibacterial activity, while they have negligible toxicity for both the human body and the environment [xii,xiii]. Their antibacterial activity is mainly due to the amino groups they contain, since positively charged groups can interact with negatively charged bacterial phospholipid membranes [xiv,xv]. However, the exact mechanism of carbon based nanomaterials antibacterial activity is more complicated and remains obscure. It should also be noted that in all cases in the literature the minimum concentration of the hybrid-Carbon based materials showing sufficient antibacterial action, is much larger, i.e. about ten times greater, than the corresponding concentration of the materials described for the first time in this invention,

Due to the enhanced antibacterial properties of carbon-based nanomaterials, research activities have recently focused on their toxicity on photosynthetic organisms, investigating the possible impact of these materials in photosynthetic pathways.

In photosynthetic organisms, unlike bacteria, a favorable effect of MWCNTs and graphene oxide was observed in the fertilization and development of cereals [xvi,xvii]. Additionally, recent studies on the influence of CNTs on algae revealed that the nanomaterials did not affect photosynthesis and any negative effects on their development were due to turbidity resulting in reduction of the available light. [xvii]. Furthermore, beneficial effects of CNTs on photosynthetic organisms were observed attributed to their anti-oxidant properties, quenching the harmful oxygen free radicals produced during photosynthesis [xix].

Photosynthesis, i.e. the conversion of light energy into chemical energy, is one of the most important biological processes of the planet. Organisms with photosynthetic ability are called phototrophic or photoautotrophic. Photosynthesis is characterized by two distinct groups of reactions: the light reactions, in which the light energy is saved as chemical energy, while producing molecular oxygen ($O_2$) as a byproduct, and the dark reactions, in which chemical energy is utilized for the reduction of $CO_2$ towards organic compounds [xx]. The oxygen producing photosynthesis, the main converter of solar energy into chemical, is catalyzed by four protein complexes of four subunits. Specifically, at the thylakoid membranes of chloroplasts, $H_2O$ is oxidized, NADP (Nicotinamide adenine dinucleotide phosphate) is reduced and ATP (adenosine triphosphate) is synthesized by two photosystems [(photosystem II (referred to herein as PS II) and photosystem I (referred to herein as PS I)], a complex of cytochrome (b6f) that mediates the transfer of electrons from PS II to PS I as well as a F-ATPase that converts the energy generated by electron transfer reactions and redox potential in the proton ATP, through Proton Motive Force (hereinafter referred to as PMF) [xxi].

Several inhibitors of PS II, mainly preventing electron transfer, are referred in the literature including some natural benzoquinones, e.g. 3-(3,4-dichlorophenyl)-1,1-dimethylurea (DCMU), as well as other compounds acting as herbicides, such as atrazine and metribuzin [xxii, xxiii].

On the contrary, no substance has ever been reported to completely inhibit the functions of PS I.

The Nanomaterials described for the first time in the present invention are the first substances capable to inhibit PS I. Moreover, their behavior is so successful that inhibition is almost complete, reaching almost 100 %.

On the other hand, the methods for determination of antibacterial action of various substances can be categorized on the basis of the results they give, in qualitative methods and quantitative methods [xxiv, xxv]. Especially for textiles, such as fabrics, technical textile etc., the main quality standards for the determination of their antibacterial properties are: AATCC 147: 2004 (American Association of Textile Chemists and Colorists), ISO 20645: 2004, SN 195920: 1992 (International Standards Organization) and JIS L 1902: 2002 - Halo method (Japanese Industrial Standard), while the main standards for quantification measurements are: AATCC 100: 2004, JIS L 1902: 2002 - Absorption method, SN 195924-1992 and ISO 20743: 2007 [xxvi, xxvii, xxviii].

The standard methodologies for the qualitative determination of the antibacterial activity of various substances are based on the inhibition of bacterial growth on agar around the test sample under examination, but they do not provide any measurement of the number of bacteria or their colonies. However, this type of approach is not applicable to a wide variety of cases, in which certain antibacterial substances, such as quaternary silanes or PHMB (poly(hexamethylene) biguanide hydrochloride), interact with the agar. Additionally, as the determination of the substance's activity is based on the degree of diffusion in the agar surrounding the sample, this method cannot be used for comparison of different substances, since each one has different diffusion behavior. Furthermore, the result is also influenced by the weight of the test material, since the amount of the antibacterial expressed as % by weight of sample. Therefore, a sample with low specific weight may give false negative results, although it has antibacterial properties. Finally, most methods for the determination of antibacterial properties cannot be implemented under the actual conditions of the materials intended use [xxix].

In quantitative methods, the antibacterial activity of a substance is expressed by the percentage of remaining bacterial population. The material that contains the substance under investigation is initially inoculated with known amount of bacteria and allowed to incubate. The bacteria are subsequently eluted from the inoculated samples and after successive dilutions and incubation on plates containing growth medium the resulting colonies are counted, compared with the control and the percent reduction of bacterial population is calculated [xxx]. The main drawback of this methodology is that after incubation of the bacteria on the surface under study, bacteria are re-incubated in ideal conditions (excess nutrient and carefully adjusted conditions of temperature and humidity), which conditions are far from the actual application conditions of antibacterial material. The quantitative methods although they are more accurate and more reliable than qualitative ones, they require more time and cost, are more difficult and complicated and quite dependent on the analyst. Thus, the results obtained from different laboratories often differ significantly [xxxi]. As evident, the above mentioned methods of determining the antibacterial activity exhibit several disadvantages which render them problematic.

The current invention proposes a novel and effective method for the quantitative assay of the antibacterial activity of substances that can be applied to any type of material under conditions realistic for the material.

**Brief description of invention**

The present invention deals with the development of novel nanocomposite hybrid materials based on dendritic polymer-functionalized nanostructured carbon-based materials. In the present invention, when dendritic polymers are mentioned, we are referring to dendrimers or hyperbranched polymers. The novel hybrid nanocomposite materials of the present invention are capable to yield stable aqueous dispersions and exhibit enhanced antibacterial properties. They can be used on various surfaces, e.g. in the preparation of textile materials, wood, metal, synthetic or other composite surfaces, etc. giving them antibacterial properties.

Additionally, the present invention deals with the mechanism of action of these hybrid materials on photosynthesis. Specifically, hybrid materials with antibacterial properties are obtained by electrostatic interactions of positively charged dendritic polymers with nanostructured carbon-based materials. The effect of these novel materials on photosynthesis of, for example, cyanobacteria and spinach thylakoid membranes is unique due to its specificity (different inhibition of photosystem I, and photosystem II) and specifically due to the fully inhibitory effect on photosystem I (PS I).

Furthermore, the present invention deals with an in situ, direct and instrumental method for the evaluation and assessment of antibacterial properties applicable on various materials (organic, inorganic, natural or synthetic) and surfaces based on measurements of Chl $\alpha$ fluorescence in gram(-) cyanobacterium *Synechococcus* sp.. The proposed procedure, employs photosynthetic gram(-) cyanobacteria deposited directly onto the surface under study and assesses cell proliferation and viability via a quick, accurate and reproducible, instrumental chlorophyll fluorescence spectrophotometric measurement. Specifically, in this method the fluorescence value of chlorophyll a at zero time after dark acclimation ($F_0$) is used to measure the increase or decrease of cyanobacteria population. Cyanobacteria, used as the test organism for the Antimicrobial Susceptibility Testing (AST) method described in this invention, are gram(-) bacteria, hence, effects of antibacterial activity on cyanobacteria can be projected to gram(-) bacteria in general. In the present invention, an example is presented using wool as model substrate, but it should be noted that this method can be applied to determine the bacterial protection of any material, such as metal, wood, glass, plastic, thermoplastics, synthetic or composite material, textile (woven and nonwoven), among others. The main advantages of this method are its low application costs and the potential of simultaneous evaluation of many different samples. However, further advantages include: i) measurements are performed while bacteria are in contact with the surface under study throughout the whole duration of the experiments without requiring special conditions for growth ii) the method can be applied under conditions identical to those of the material's intended use. Additionally, in contrast with existing methods of determination of antibacterial properties, it produces high resolution and quantitative results and is so versatile that it could be used to evaluate the antibacterial properties of any compound (organic, inorganic, natural or man-made) under any experimental conditions, depending on the targeted application.

In a preferred application, the invention describes the development of hybrid nanocomposite materials which are obtained by the electrostatic interaction of dendritic polymers with symmetrical or asymmetrical architecture, i.e., dendrimers or hyperbranched polymers, functionalized with positively charged groups with nanostructured carbon-based materials containing negatively charged moieties. Preferably, the positively charged groups of hybrid nanocomposite materials described in this invention are guanidinium moieties or quaternary ammonium groups or both or any other positively charged moieties which can be attached at dendritic scaffold.

Advantageously, in the hybrid nanocomposite materials described of the invention the nanostructured carbon-based materials are oxidized carbon nanotubes (f-CNTs) or graphene oxide (GO) or oxidized carbon nanodisks (f-CNDs) or/and all together. Advantageously, the minimum concentration of functional dendritic polymer in hybrid nanocomposite materials, described in this invention, is 5%.

Preferably, the hybrid nanocomposite materials of the invention exhibit antibacterial properties starting from concentrations as low as 5 $\mu g \cdot mL^{-1}$.

It is especially advantageous that the hybrid nanocomposite materials of the invention exhibit complete inhibition (up to 98%) of the activity of photosystem I. The hybrid nanocomposite materials of the invention exhibit at least 50% inhibition of the activity of Photosystem II.

Preferably, the hybrid nanocomposite materials of the invention comprise functionalized dendritic polymers with symmetrical or asymmetrical architecture, derived from commercially available dendritic polymers such as:

- ✔ Hyperbranched polyethylenimine with molecular weight 400-750.000 Da,
- ✔ Diaminobutane propyleneimine dendrimers (DAB) with molecular weight 200-7.000 Da,
- ✔ Poly(amido amine) (PAMAM) dendrimers with molecular weight 200-150.000 Da,
- ✔ other dendritic polymers exhibiting similar characteristics

which will be functionalized with positively charged groups at 10-100% molecular coverage.

Particular advantages are presented by the method of preparing hybrid nanocomposite materials of the invention. The method comprises the following steps:

- ✔ Dispersion in water or other suitable solvent with the aid of ultrasound of one part of each nanostructured carbon-based material and subsequent addition of 3 parts of functionalized dendritic polymers with symmetric or asymmetric architecture, dissolved in the same solvent, bearing positively charged groups,
- ✔ Ultrasound of the resulting dispersions and then stirring; advantageously stirring is for 12 hours,
- ✔ Ultracentrifugation and washing with appropriate solvent at least once, preferably three times,
- ✔ Lyophilization or evaporation of solvent in order to obtain the final hybrids in the form of powder.

Preferably, a method according to the invention for the production of textile material (woven and nonwoven), metal, wood, glass, plastics, thermoplastics and other synthetic or composite material with bacterial protection which is prepared by the incorporation or coating of the nanocomposites hybrid materials presented in the current invention to above mention substrate.

Preferably, in the method of the present invention the lowest loading of hybrid nanocomposite materials of the invention in textile material (woven and nonwoven), metal, wood, glass, plastics, thermoplastics and other synthetic or composite material to achieve fully antibacterial properties, is 1% w/w.

Additionally, the present invention also describes a quantitative method for the evaluation and assessment of antibacterial properties of any compound (organic, inorganic, natural or man-made) incorporated to any material or substrate (polymers, metal, wood, woven and nonwoven textile materials, synthetic or composite materials, plastics, thermoplastics, etc.) under any experimental conditions, depending on the targeted application is described. Specifically, this method:

- ✔ correlates the fluorescence value ($F_o$) of chlorophyll $\alpha$ (Chl $\alpha$) of cyanobacteria at zero time after dark acclimation ($F_o$) with the increase or decrease of their population and
- ✔ quantitatively determines the degree of bacterial protection in conditions analogous to that of the intended use of each material or substrate.

It is advantageous that in the aforementioned quantitative determination method of antibacterial properties of any compound (organic, inorganic, natural or man-made) incorporated to any material or substrate according to this invention, the following steps are comprised:

✔ incorporation of hybrid nanocomposite materials of the present invention, or any other material that exhibits antibacterial properties to the substrate / material (any),

✔ incubation of cyanobacteria directly on the material,

✔ measurement of $F_0$ on the material,

✔ determination of the growth of bacteria on the material based on the value of fluorescence measured at specified intervals and from the change of fluorescence value,

✔ increase of the measured fluorescence value that is due to the growth of bacteria population leading to the conclusion that the material does not exhibit antibacterial properties, while if the fluorescence value is low, stable, or decreasing, the material exhibits antibacterial properties.

The invention is described with reference to the following examples and tables and schemes presented below, which should be taken into account illustratively and in explanation and not restrictively.

**Brief description of Figures**

Figure 1 shows: Growth curve of cyanobacterium *Synechococcus* sp. PCC7942 in the presence of G-PEI-5K

Figure 2 shows: Growth curve of cyanobacterium *Synechococcus* sp. PCC7942 in the presence of G-PEI-25K

Figure 3 shows: Growth curve of cyanobacterium *Synechococcus* sp. PCC7942 in the presence of f-CNTs/G-PEI-5K,

Figure 4 shows: Growth curve of cyanobacterium *Synechococcus* sp. PCC7942 in the presence of f-CNTs.

Figure 5 shows: Growth curve of cyanobacterium *Synechococcus* sp. PCC7942 in the presence of GO/G-PEI-5K.

Figure 6 shows: Growth curve of cyanobacterium *Synechococcus* sp. PCC7942 in the presence of GO/G-PEI-25K.

Figure 7 shows: Growth curve of cyanobacterium *Synechococcus* sp. PCC7942 in the presence of GO.

Figure 8 shows: Growth curve of cyanobacterium *Synechococcus* sp. PCC7942 in the presence of f-GNDs/G-PEI-5K.

Figure 9 shows: Growth curve of cyanobacterium *Synechococcus* sp. PCC7942 in the presence of f-CNDs/G-PEI-25K.

Figure 10 shows: Growth curve of cyanobacterium *Synechococcus* sp. PCC7942 in the presence of f-CNDs.

Figure 11 shows: Growth curve of cyanobacterium *Synechococcus* sp. PCC7942 in the presence of f-CNTs/30-G-PEI-25K.

Figure 12 shows: Growth curve of cyanobacterium *Synechococcus* sp. PCC7942 in the presence of f-CNTs/50-G-PEI-25K.

Figure 13 shows: Growth curve of cyanobacterium *Synechococcus* sp. PCC7942 in the presence of f-CNTs/80-G-PEI-25K.

Figure 14 shows: Growth curve of green algae *C. reinhardtii* in the presence of G-PEI-5K

Figure 15 shows: Growth curve of green algae *C. reinhardtii* in the presence of G-PEI-25K

Figure 16 shows: Growth curve of green algae *C. reinhardtii* in the presence of f-CNTs/G-PEI-5K.

Figure 17 shows: Growth curve of green algae *C. reinhardtii* in the presence of f-CNTs.

Figure 18 shows: Growth curve of green algae *C. reinhardtii* in the presence of GO/G-PEI-5K.

Figure 19 shows: Growth curve of green algae *C. reinhardtii* in the presence of GO/G-PEI-25K.

Figure 20 shows: Growth curve of green algae *C. reinhardtii* in the presence of GO.

The Figure 21 shows: Growth curve of green algae *C. reinhardtii* in the presence of f-CNDs/G-PEI-5K.

The Figure 22 shows: Growth curve of green algae *C. reinhardtii* in the presence of f-CNDs/G-PEI-25K.

The Figure 23 shows: Growth curve of green algae *C. reinhardtii* in the presence of f-CNDs.

The Figure 24 shows: Growth curve of green algae *C. reinhardtii* in the presence of f-CNTs/30-G-PEI-25K.

The Figure 25 shows: Growth curve of green algae *C. reinhardtii* in the presence of f-CNTs/50-G-PEI-25K.

The Figure 26 shows: Growth curve of green algae *C. reinhardtii* in the presence of f-CNTs/80-G-PEI-25K.

The Figure 27 shows: Relative absorbance/reflectance curves at 820 nm induced by far-red light of cyanobacterial permeaplasts in the presence of G-PEI-5K, f-CNTs καl f-CNTs/G-PEI-5K.

The Figure 28 shows: Relative absorbance/reflectance curves at 820 nm induced by far-red light of spinach thylakoid membranes in the presence of G-PEI-5K, f-CNTs καl f-CNTs/G-PEI-5K.

The Figure 29 shows: Chl $\alpha$ fluorescence changes ($F_0$) of cyanobacterium *Synechococcus* sp. PCC7942 adapted on textile in the presence of f-CNTs/G-PEI-5K.

The Figure 30 shows: Chl $\alpha$ fluorescence changes ($F_0$) of cyanobacterium *Synechococcus* sp. PCC7942 adapted on textile in the presence of f-CNTs/G-PEI-25K

The Figure 31 shows: Chl $\alpha$ fluorescence changes ($F_0$) of cyanobacterium *Synechococcus* sp. PCC7942 adapted on washed textile in the presence of 2% f-CNTs/G-PEI-5K (w/w).

The Figure 32 shows: $M_i$, evolution curves of cyanobacteria on HDPE, HDPE/G-PEI-5K and HDPE/G-PEI-5K/f-CNTs nanocomposites surfaces.

The Figure 33 shows: $M_i$ evolution curves of cyanobacteria on HDPE, HDPE/G-PEI-5K and HDPE/G-PEI-5K/f-CNTs nanocomposites holes.

**Brief description of Picture**

The Picture 1 shows: Aqueous dispersions (10 mg/mL) of f-CNTs/G-PEI-5K (right), f-CNTs/50-Q-PEI-25K (left), after one year at 25 oC.

**Detailed description of the invention and examples**

**Examples of synthetic routes and methods**

Examples of synthetic routes of nanocomposite hybrid materials based on dendritic polymers, functionalized with positively charged groups (e.g. guanidinium or quaternary ammonium groups) and nanostructured carbon-based materials, i.e. oxidized carbon nanotubes, graphene oxide and oxidized carbon nanodisks, containing negatively charged moieties, are mentioned below.

Initially we describe the syntheses of functionalized dendritic polymers and then describe the preparation method of the hybrid nanocomposite materials.

**Synthesis of guanidinylated derivatives of polyethyleneimine**

The guanidinylated derivatives of polyethyleneimine were prepared with analogous method described in the literature [xxxii]. Specifically, one part of hyperbranched polyethyleneimine (PEI) with molecular weight 5000 Da ή 25000 Da, dissolved in dimethylformamide (DMF), was added to a DMF solution containing 20 ή 200 parts of 1H-pyrazole-1-carboxamidine hydrochloride and 80 ή 400 parts *N,N*-diisopropylethylamine, respectively. The mixture was allowed to react at room temperature for 24h under argon atmosphere. The products were precipitated with diethyl ether and the crude products were dissolved in water and were subjected to dialysis (mol. weight cut-off: 1200). The final products, G-PEI-5K καὶ G-PEI-25K, were received after lyophilization. The successful attachment of guanidinium groups to PEI scaffold and the degree of guanidinylation were established using NMR spectroscopy. The degree of substitution of both products is found to be 50%.

$^1$H NMR: (500 MHz, DMSO-d$_6$) $\delta$ (ppm) = 7.85 (broad s, NH), 7.20 (broad s, NH$_2^+$), 3.15 (m, NCH$_2$CH$_2$NH-G), 2.70 - 2.50 (m, CH$_2$ of PEI scaffold).

$^{13}$C NMR (125.1 MHz, D2O): $\delta$ (ppm) = 158.5 (CH$_2$NHC(NH$_2$)NH$_2^+$), 55-51.0 (CH$_2$ of PEI scaffold), 42.0 καὶ 40.0 (CH$_2$NH$_2$ primary and secondary, respectively), 39 καὶ 36 (CH$_2$NHC(NH$_2$)NH$_2^+$ + primary and secondary, respectively).

**Synthesis of quaternized derivatives of polyethyleneimine**

The quaternized derivatives of polyethyleneimine were prepared with analogous method described in the literature [xxxiii]. Specifically, quaternized derivatives of dendritic polyethyleneimine with different degree of substitution were prepared. The synthetic method is as follows: one part of hyperbranched polyethyleneimine (PEI) with molecular weight 25000 Da, was dissolved in an aqueous solution containing 80 or 400 parts of triethylamine. Subsequently, analogous parts of glycidyltrimethylammonium chloride are added to the mixture in order to receive the final quaternized derivatives with degree of substation 30% (30-Q-PEI-25K), 50% (50-Q-PEI-25K) and 80% (80-Q-PEI-25K). The mixture was allowed to react for several hours at room temperature and subsequently subjected to dialysis with the appropriate membrane. The final products, 30-Q-PEI-25K, 50-Q-PEI-25K and 80-Q-PEI-25K, were received after lyophilization. The introduction of the quaternary moieties at the external surface of the parent PEI was confirmed by NMR spectroscopy. Additionally, the degree of quaternization was calculated by the integration of peaks at 3.45 ppm and 270-2.50 ppm in the NMR spectra.

$^1$H NMR: (500 MHz, D$_2$O) $\delta$ (ppm) = 4.25 (broad s, CH-OH), 3.45 (m, CH$_2$N(CH$_3$)$^+$), 3.25 (CH$_3$), 2.70 - 2.50 (m, CH$_2$ of PEI scaffold).

$^{13}$C NMR (125.1 MHz, D$_2$O): 5 (ppm) = 71.5 (CH$_2$N(CH$_3$)$^+$), 67.5 (CH-OH), 57.0 (CH$_3$), 55-51.0 (CH$_2$ of PEI scaffold), 51.0 καὶ 48.0 (CH$_2$NH-Q primary and secondary, respectively), 42.0 καὶ 40.0 (CH$_2$NH$_2$ primary and secondary, respectively).

**Preparation of dendritic polymer-functionalized nanostructured carbon-based materials**

For the preparation of hybrid nanocomposite materials, oxidized carbon nanotubes (f-CNTs), graphene oxide (GO) and oxidized carbon nanodisks (f-CNDs) as well as the above mentioned dendritic derivatives were used. Specifically, one part of oxidized carbon nanotubes (f-CNTs) or graphene oxide (GO) or oxidized carbon nanodisks (f-CNDs) is dispersed in water (20mL) with the aid of ultrasound (200Hz) and subsequently 3 parts of functionalized dendritic polymers dissolved in water are added. The aqueous dispersions are subjected to ultrasound for a further 15 min and then left under stirring for 12 hours. In order to remove the excess of polymeric derivatives, the aqueous dispersions are ultra-centrifuged at 45.000 rpm for 45 min and the products are washed with water three times. The final hybrid nanocomposites (f-CNTs/G-PEI-5K, f-CNTs, GO/G-PEI-5K, GO/G-PEI-25K, GO, f-CNDs/G-PEI-5K, f-CNDs/G-PEI-25K, f-CNDs, f-CNTs/30-Q-PEI-25K, f-CNTs/50-Q-PEI-25K and f-CNTs/80-Q-PEI-25K) are received after lyophilization in form of powder. The produced materials are physico-chemically characterized using a variety of techniques such as FTIR, XPS, RAMAN, X-ray diffraction, TGA, SEM, TEM, etc. The loading of functional dendritic polymer in hybrid nanocomposite materials is 10 to 20% w/w for materials containing f-CNTs, 20 to 30% w/w for materials containing graphene oxide (GO) and 25 to 30% w/w for materials containing oxidized nanodisks, as revealed by elemental analysis and TGA.

Aqueous dispersions obtained by addition of the prepared materials in water followed by ultra-sonication are stable for at least one year at 25 °C, even at concentrations of 10 mg / mL as presented in Picture 1 showing aqueous dispersions (10 mg / mL) of f-CNTs / G-PEI-5K (right), f-CNTs / 50-Q-PEI-25K (left), after one year at 25 °C.
The prepared materials were subsequently evaluated as antibacterial agents.

## 1. Culture of cyanobacterium Synechococcus sp. PCC7942

The unicellular cyanobacterium Synechococcus sp. PCC7942, was cultured in BG11 [xxxiv,xxxv] containing 20 mM HEPES, pH 7.5 (basal medium). The culture media was sterilized at 121 °C. The cultures were incubated under white fluorescent light (100 $\mu$E·m$^{-2}$·s$^{-1}$), in an orbital incubator at 31 °C [xxxvi]. The determination of the concentration of chlorophyll a (Chl $\alpha$) of cyanobacteria over time, is an indicator of cyanobacteria growth. The ion-permeable cyanobacteria cells (permeaplastes) were prepared by partial hydrolysis of the peptidoglycan of bacteria walls after their treatment with lysozyme [xxxvii,xxxviii]. Culture growth was monitored in terms of concentration of Chl $\alpha$, determined in N,N-dimethylformamide extracts of cell pellets [xxxix].

## 2. Algae Growth

We used the unicellular green alga *Chlamydomonas reinhardtii* as the test organism. C. *reinhardtii* was cultured in the medium TAP [xi].Cell proliferation was estimated in terms of total chlorophyll concentration, in $\mu$g (Chl $\alpha$ + Chl b) ml$^{-1}$ [39].

## 3. Isolation of thylakoids from spinach leaves

Mature spinach leaves used to isolate thylakoid membranes according to Chrysina et al. [xii].Thylakoid membranes were resuspended in a medium containing 20 mM MES-NaOH, 400 mM sucrose, 10 mM NaCl, 20 mM KCl, 2 mM MgCl$_2$ (pH 6.5) in H$_2$O and the suspension was stored at -80 °C until use. Total Chl concentration was calculated as previously [39].

## 4, Antibacterial effect of polyethyleneimine derivatives and of carbon-based hybrid materials on the proliferation of cyanobacterium *Synechococcus* sp. PCC7942 and green algae *C. reinhardtii.*

Studies on the growth of *Synechococcus* sp. PCC7942 in the presence of G-PEI-5K, G-PEI-25K, f-CNTs/G-PEI-5K, f-CNTs, GO/G-PEI-5K, GO/G-PEI-25K, GO, f-CNDs/G-PEI-5K, f-CNDs/G-PEI-25K, f-CNDs, f-CNTs/30-Q-PEI-25K, f-CNTs/50-Q-PEI-25K and f-CNTs/80-Q-PEI-25K were carried out.

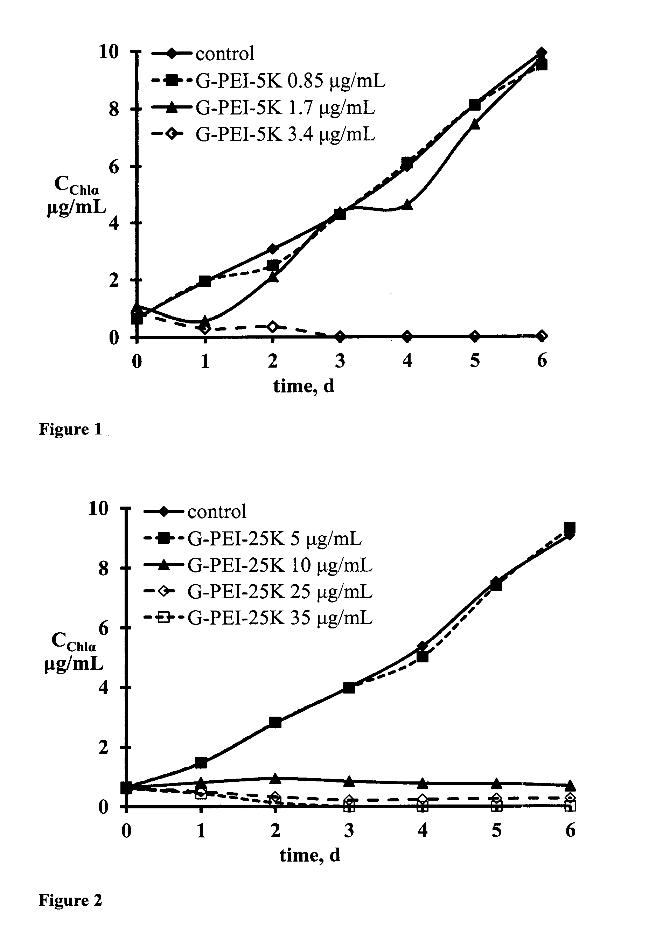

Figure 1 shows plots of the proliferation of cyanobacteria against culture time in the presence of G-PEI-5K. As clearly shown, the proliferation of cyanobacterial cells is not inhibited at 1.7$\mu$g/ml, while growth is inhibited at, or above, 3.4$\mu$g/ml. Figure 2 shows cyanobacterial proliferation curves in the presence of G-PEI-25K. Cell proliferation is not inhibited at 5$\mu$g/ml, while it is inhibited at 10$\mu$g/ml and it is toxic above 35$\mu$g/ml. In conclusion, G-PEI-5K and G-PEI-25K do penetrate into the cyanobacterial cells and fully inhibit their proliferation at 3.4$\mu$g/ml and at 35$\mu$g/ml, respectively. Moreover, the G-PEI-25K, with 5 times higher molecular weight than the G-PEI-5K, cannot easily penetrate into the cell. Thus its effective concentration is 10 times higher compared to that of G-PEI-5K.

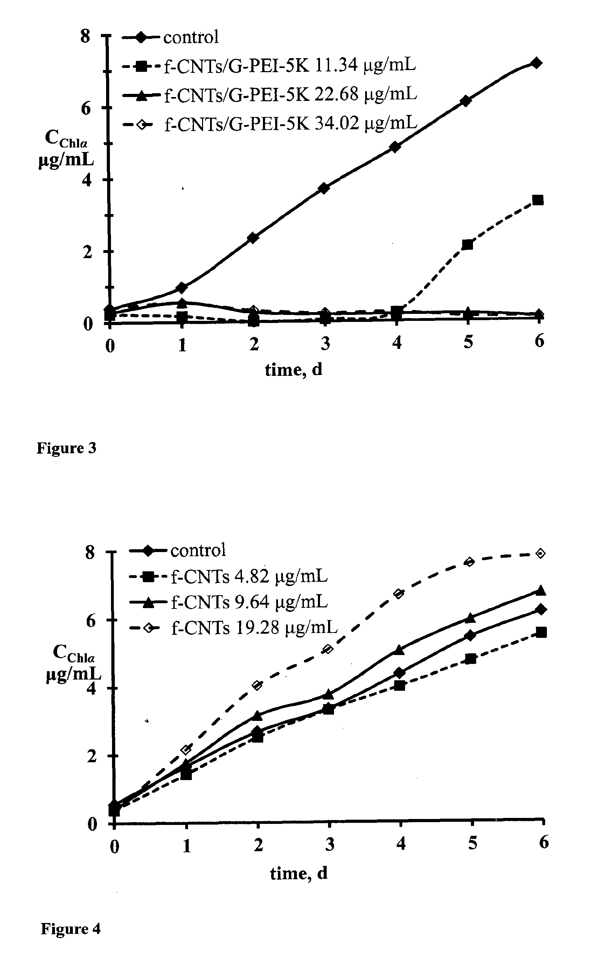

Figure 3 shows the time trace of cyanobacteria proliferation in the presence of the f-CNTs/G-PEI-5K hybrid material. f-CNTs/G-PEI-5K penetrates into the cells and inhibits their proliferation at 22.68 $\mu$g/ml, while at concentration

of 11.34 $\mu$g/ml there is a lag phase, in their growth until the 4th day.

In order to investigate the effect of pure polymers or CNTs/polymer hybrids in cell proliferation, a new series of experiments were performed. Figure 4 shows the time trace of cyanobacteria proliferation in the presence of f-CNTs. f-CNTs do not inhibit cyanobacterial proliferation suggesting that either they cannot penetrate into cells or they do not inhibit photosynthesis.

Therefore all the above results clearly demonstrate the antibacterial properties of G-PEI-5K, G-PEI-25K and f-CNTs/G-PEI-5K.

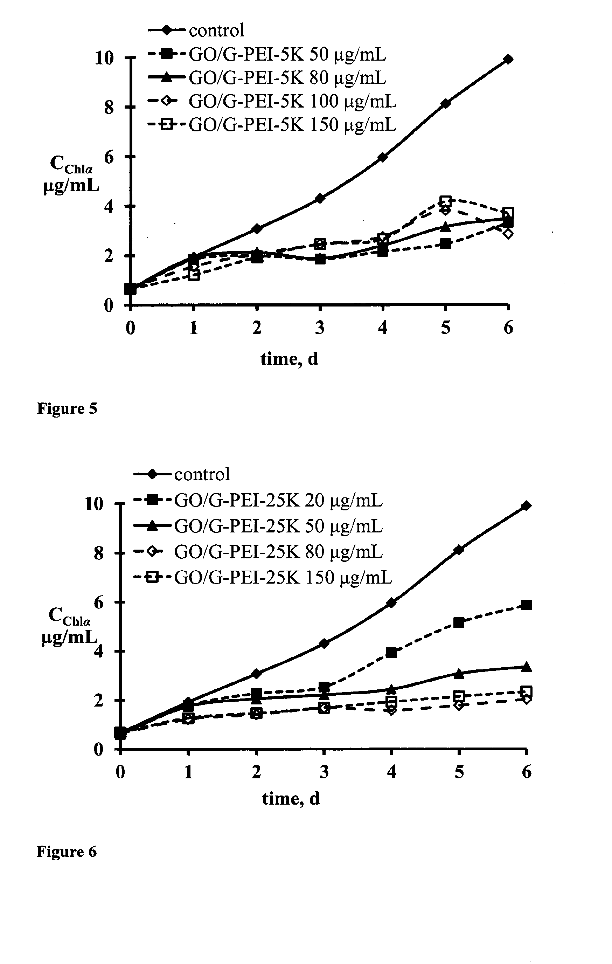

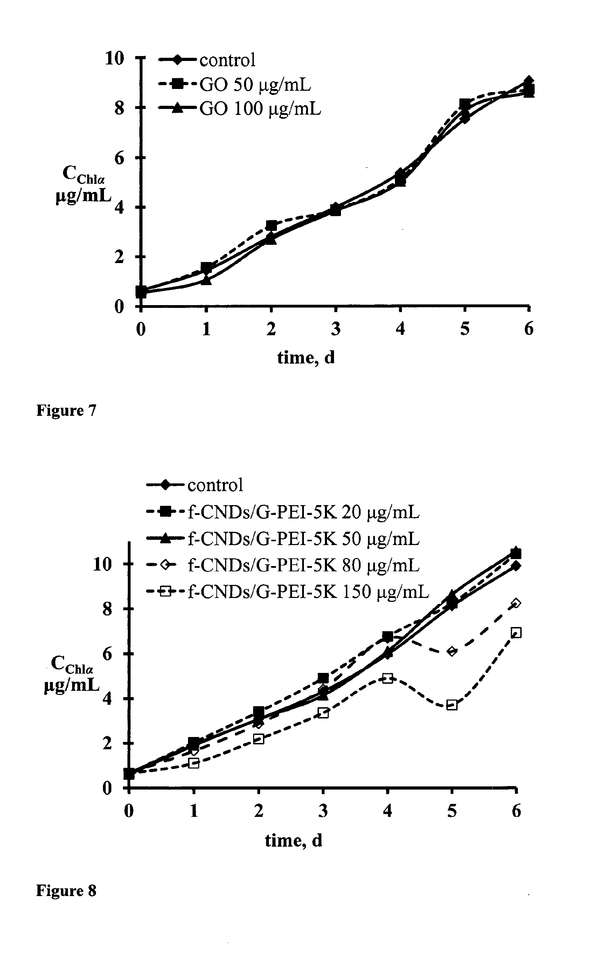

Figures 5 and 6 show the time trace of cyanobacteria proliferation in the presence of the GO/G-PEI-5K and GO/G-PEI-25K hybrid materials. Even though cell proliferation is inhibited, at these concentrations of GO/G-PEI-5K and GO/G-PEI-25K, the hybrids are not bacteriocidal. Moreover, Figure 7 shows that GO do not inhibit cyanobacterial proliferation.

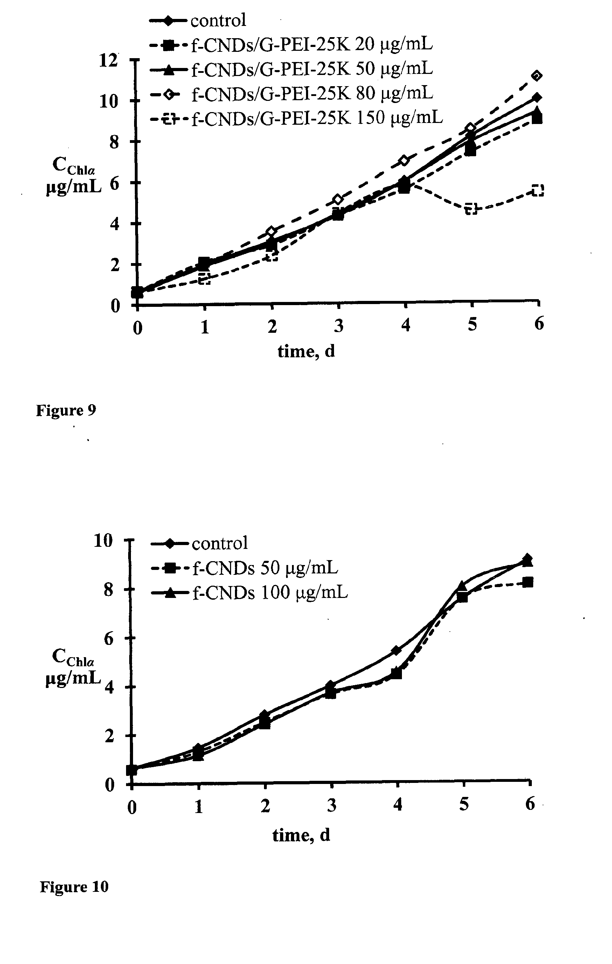

Figures 8, 9 and 10 show time traces of cyanobacteria proliferation in the presence of f-CNDs/G-PEI-5K, f-CNDs/G-PEI-25K, and f-CNDs. f-CNDs/G-PEI-5K, f-CNDs/G-PEI-25K, and f-CNDs do not inhibit cell proliferation at the corresponding concentrations.

We can conclude then, that GO/G-PEI-5K, GO/G-PEI-25K, GO, f-CNDs/G-PEI-5K, f-CNDs/G-PEI-25K and f-CNDs, exhibit decreased antibacterial properties, leading to cyanobacteria proliferation, at the studied concentrations.

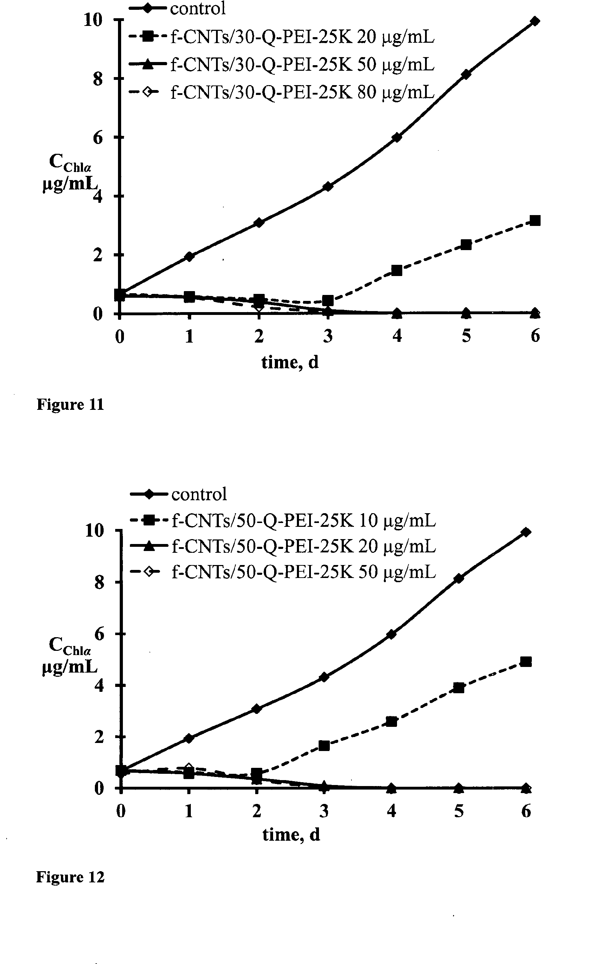

Figures 11, 12 and 13 show time traces of cyanobacteria proliferation in the presence of f-CNTs/30-Q-PEI-25K, f-CNTs/50-Q-PEI-25K, and f-CNTs/80-Q-PEI-25K hybrid materials, respectively. f-CNTs/30-Q-PEI-25K, f-CNTs/50-Q-PEI-25K, and f-CNTs/80-Q-PEI-25K inhibit significantly cyanobacterial growth at 20 $\mu$g/ml, 10 $\mu$g/ml and 10 $\mu$g/ml respectively. Moreover, the minimum concentration, at which the proliferation inhibition is complete, is 20 $\mu$g/ml for both f-CNTs/50-Q-PEI-25K and f-CNTs/80-Q-PEI-25K, while for f-CNTs/30-Q-PEI-25K is 50 $\mu$g/ml.

In conclusion, f-CNTs/30-Q-PEI-25K, f-CNTs/50-Q-PEI-25K, and f-CNTs/80-Q-PEI-25K reveal significant antibacterial properties.

### *C. reinhardtii* cell proliferation

Our results in the antibacterial effect of the above hybrid materials on cyanobacteria proliferation led us to study their effects on the proliferation of green algae. Green algae are eukaryotes and their photosynthetic machinery is similar to that of the higher plants.

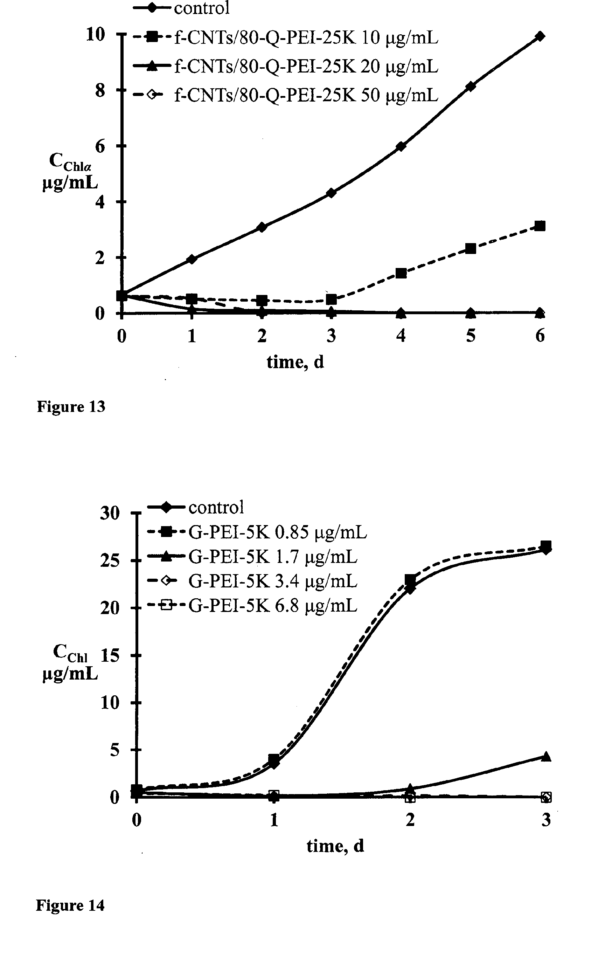

Figure 14 shows green alga C. *reinhardtii* cell proliferation curves in the presence of G-PEI-5K. Cell proliferation is not inhibited up to 0.85$\mu$g/ml, while 1.7$\mu$g/ml is the concentration threshold of cells' proliferation. At higher concentrations (above 3.4 $\mu$g/ml), G-PEI-5K is toxic for cells.

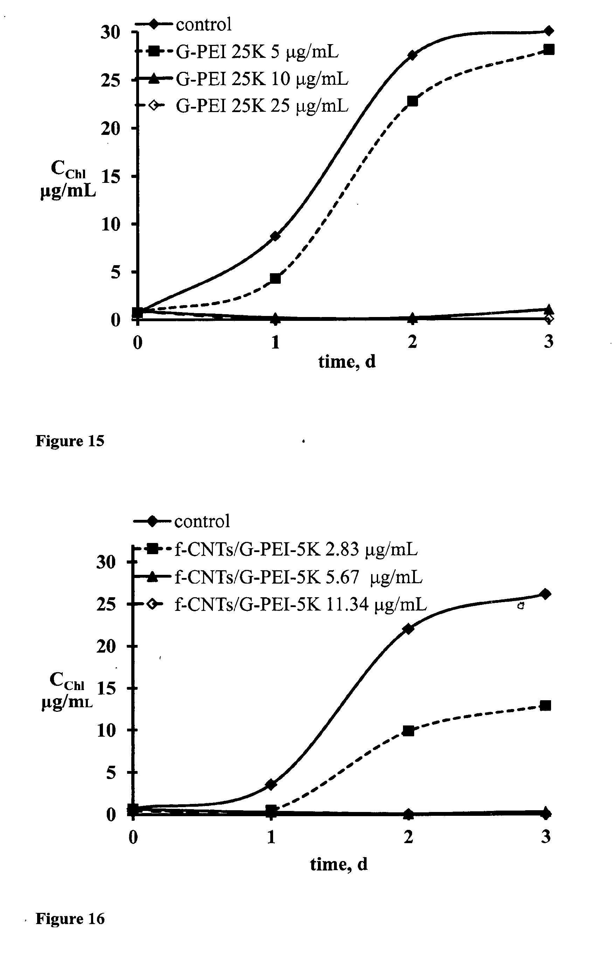

Figure 15 shows *C. reinhardtii* cell proliferation curves in the presence of G-PEI-25K, Cell proliferation is not inhibited up to concentration of 5.0$\mu$g/ml, while C-PEI-25K exhibits cell toxicity at concentration above 35.0$\mu$g/ml. Figures 14 and 15 clearly indicate that G-PEI-5K is more effective than G-PEI-25K. Thus, G-PEI-25K requires 7-8 fold higher concentration compared to G-PEI-5K, to exhibit the same antibacterial efficiency.

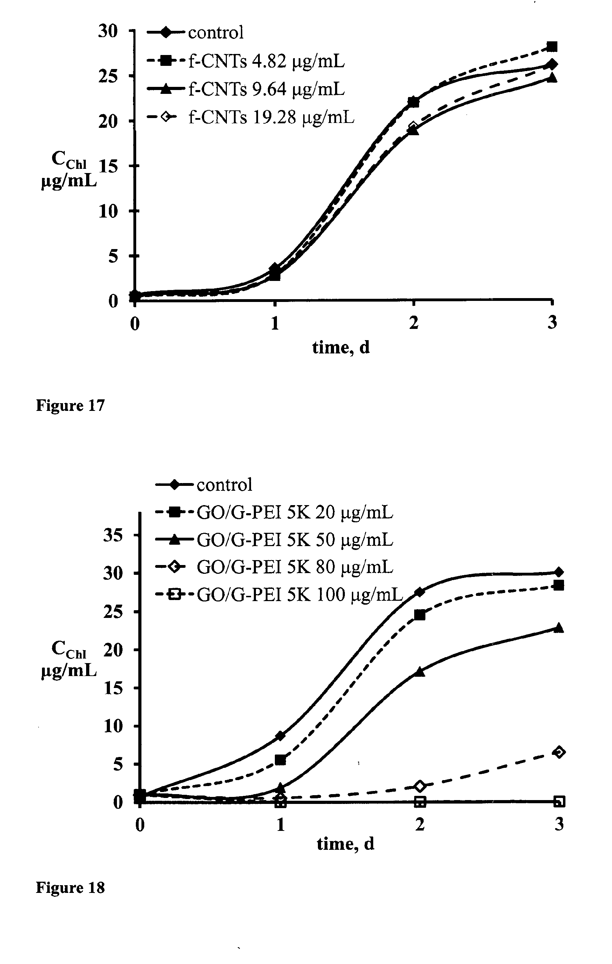

Figure 16 shows *C. reinhardtii* cell proliferation curves in the presence of f-CNTs/G-PEI-5K. Inhibition occurs at 2.83 $\mu$g/ml f-CNTs/G-PEI-5K, while at 5.67$\mu$g/ml the hybrid nanocomposite material is considered as toxic for the cells. Figure 17 clearly shows that f-CNTs do not inhibit green algae growth. This effect, which is similar with the case of cyanobacteria, can be attributed to the fact that f-CNTs either they do not penetrate into cells or they do not inhibit photosynthesis.

The above results clearly show that G-PEI-5K, G-PEI-25K as well as the hybrids f-CNTs/G-PEI-5K and f-CNTs/G-PEI-25K inhibit the growth of green algae.

Figure 18 shows C. *reinhardtii* cell proliferation curves in the presence of the hybrid material GO/G-PEI-5K. A slight inhibition of cells' proliferation is observed at concentration up to 50.0 $\mu$g/ml of GO/G-PEI-5K, while 80.0 $\mu$g/ml is the concentration threshold of cells proliferation, at which a lag phase of growth is observed. However, at concentration above the 100.0 $\mu$g/ml, GO/G-PEI-5K is completely inhibits cells growth, revealing bacteriocidal behaviour.

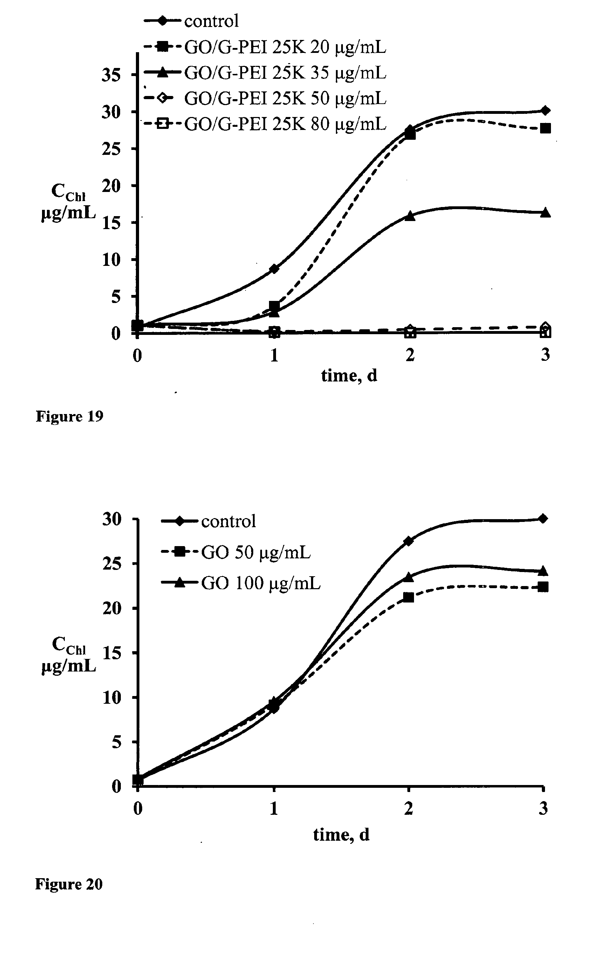

Figure 19 shows *C. reinhardtii* cell proliferation curves in the presence of the GO/G-PEI-25K. The hybrid material exhibits similar behavior with the analogous hybrid with the lower molecular weight (GO/G-PEI-5K), but it is more efficient. Specifically, the corresponding threshold of cells proliferation is 50.0 $\mu$g/ml, while at 80.0 $\mu$g/ml, GO/G-PEI-25K exhibits fully antibacterial properties. However, similarly to cyanobacteria, GO cannot inhibit the proliferation of green algae (Figure 20).

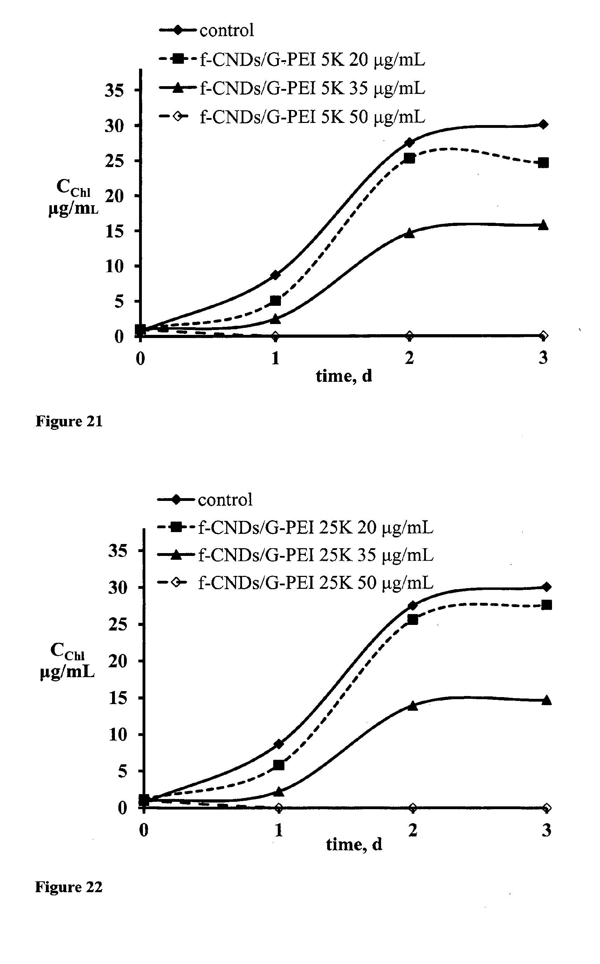

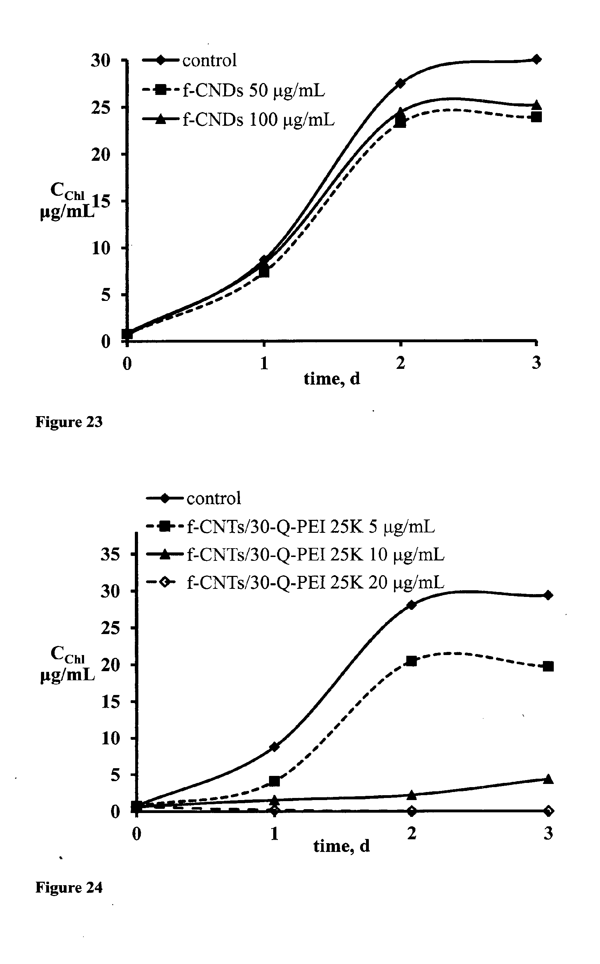

Figures 21 and 22 show *C. reinhardtii* cell proliferation curves in the presence of f-CNDs/G-PEI-5K and f-CNDs/G-PEI-25K. Both hybrid materials exhibit antibacterial properties at concentration above 50.0 $\mu$g/ml, while f-CNDs do not affect the green algae growth (Figure 23) as in the cases on f-CNTs and GO.

The abovementioned results show that GO/G-PEI-5K, GO/G-PEI-25K, f-CNDs/G-PEI-5K and f-CNDs/G-PEI-25K are toxic to green algae, preventing their growth, contrary to cyanobacteria (no inhibitory effect).

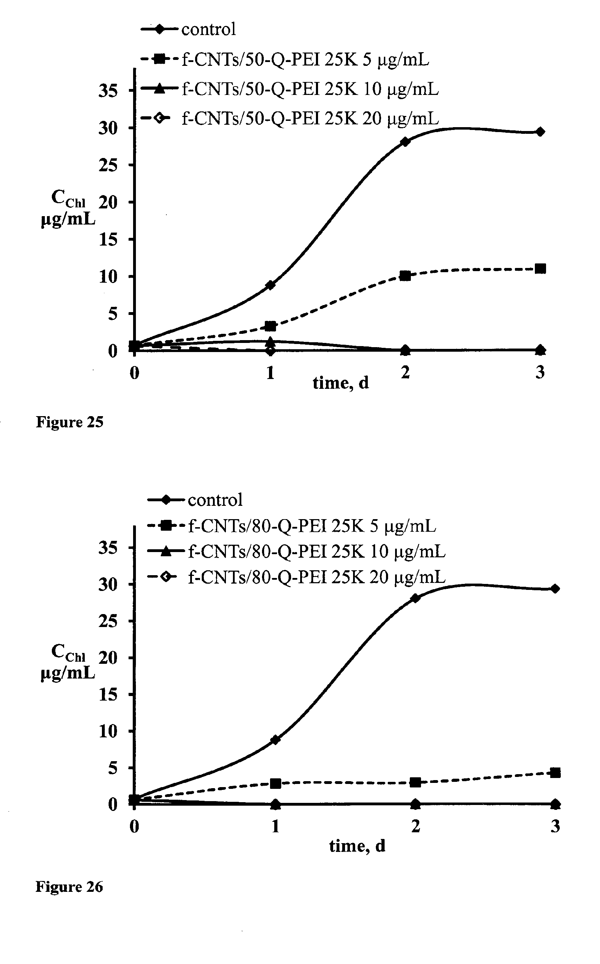

Figures 24, 25 and 26 show the C. *reinhardtii* cell proliferation curves in the presence of the f-CNTs/30-Q-PEI-25K, f-CNTs/50-Q-PEI-25K and f-CNTs/80-Q-PEI-25K hybrid materials. It appears that f-CNTs/30-Q-PEI-25K, f-CNTs/50-Q-PEI-25K, and f-CNTs/80-Q-PEI-25K are toxic for the green algae at 20μg/ml, 10μg/ml and 10μg/ml, respectively. Therefore, increasing the degree of substitution of the quaternary groups the antibacterial properties are improved. Additionally, f-CNTs/30-Q-PEI-25K, f-CNTs/50-Q-PEI-25K, and f-CNTs/80-Q-PEI-25K are more effective to green algae, than cyanobacteria.

Based on the obtained results, G-PEI-5K and f-CNTs/G-PEI-5K, which are the most effective antibacterial agents, were selected, as model systems, for further studies on the effect of hybrid nanomaterials on the photosynthesis process of cyanobacteria and green algae.

## 5. Measurement of photosynthetic activity

### a. Oxymetric Measurements of Photosystem (PS) II and PSI electron transport activities.

Photoinduced electron transport rates were determined in *Synechococcus* permeaplasts and spinach (*Spinacia olearecea*) thylakoid membranes at room temperature oxymetrically (for each Photosystem) with a Clark-type oxygen electrode (DW1; Oxygraph, Hansatech, King's Lynn, U.K.). The instrument was fitted with a slide projector to provide actinic illumination to the samples. PSII activity was monitored by measuring the rate of oxygen evolution, with water as electron donor and p-benzoquinone (1 mM) as post-PSII electron acceptor PSI activity was monitored by measuring the rate of oxygen uptake, using Na-ascorbate (2 mM)/ diaminodurene (1 mM), as electron donor to PSI and methyl viologen (0.15 mM) as post-PSI electron acceptor and mediator of oxygen uptake, in the presence of the post-PSII electron transfer inhibitor 3-(3,4-dichlorophenyl)-1,10-dimethylurea (DCMU, 20 μM) [xlii]. The reaction mixture (1 ml) contained permeaplasts (10 μg Chl a) or thylakoid membranes at 10 μg Chl.

### b. Measurement of PS I activity

The photo-oxidizable reaction center of PSI (P700$^+$) is the most reliable, accurate and direct measurement of PSI activity. The photo-oxidizable PSI pool (P700') content was measured by monitoring reflectance changes at 820 nm using a pulse amplitude modulated (PAM 101/102/103) fluorometer (Walz, Effeltrich, Germany) equipped with an ED-800T emitter and a far-red (FR-102) photo-diode detector, according to Schreiber et al. [xliii], Klughammer and Schreiber [xliv], Ivanov et al. [xlv], Wientjes and Croce [xlvi]. Reflectance was traced in the dark for 30 s and PSI was subsequently oxidized by saturating (15 W m$^{-2}$) continuous far-red light. The photo-oxidizable PSI pool P700$^+$ is estimated as $\Delta A_{820}/ A_{870}$. Then, the far-red source was turned on and reflectance was recovered to the initial value after PSI re-reduction.

### C. Studies on photosynthetic activity of cyanobacteria.

### Studies on PSII and PSI activity.

Tables 1 and 2, show the PSII and PSI activities of cyanobacterial permeaplasts, respectively. Permeaplasts were used for the penetration of methyl-viologen (MV) into the cells. Table 1 shows the effect of G-PEI-5K, f-CNTs/G-PEI-5K and f-CNTs, on the PSII activity (in μmol $O_2$ x mg Chl $\alpha^1$ x h$^{-1}$). Table 1 reveals a concentration dependent inhibition of PSII activity, with the maximum inhibition obtained in the case of f-CNTs/G-PEI-5K (~70%). The inhibitory effect follows the order: f-CNTs/G-PEI-5K >> G-PEI-5K >> f-CNTs. These results are in agreement with the inhibition of cell proliferation, for the case of f-CNTs/G-PEI-5K and G-PEI-5K, as presented earlier (see Figs 1 and 3). However, in the case of permeaplasts, f-CNTs reveal increased PSII inhibition. This result is not in line with the previously presented results (Figure 4) for cyanobacteria. The use of permeablasts allows f-CNTs to penetrate into the cells, leading to PSII activity inhibition. Based on these findings it is concluded that the poor antibacterial behavior of f-CNTs into cyanobacteria is due to their inefficiency to translocate through cell walls. However, when they enter, by means of permeablasts, they affect heavily the cells.

Table 2 shows the effect of G-PEI-5K, f-CNTs/G-PEI-5K and f-CNTs on PSI activity. f-CNTs/G-PEI-5K and f-CNTs inhibit PSI activity up to ~96% and ~97%, respectively, while G-PEI-5K inhibits PSI activity up to ~60%. The inhibition follows the order: f-CNTs/G-PEI-5K >> f-CNTs >> G-PEI-5K. This behavior is different compared to the inhibition order of PSII. As shown in Tables 1 and 2, G-PEI-5K has the similar effect on PSII and PSI activity. Moreover, most importantly, in the presence f-CNTs/G-PEI-5K and f-CNTs the PSI activity is totally inhibited (~97%), while PSII activity is inhibited up to ~70%.

**Studies on Photo- oxidative state of $P_{700}$ in cyanobacteria**

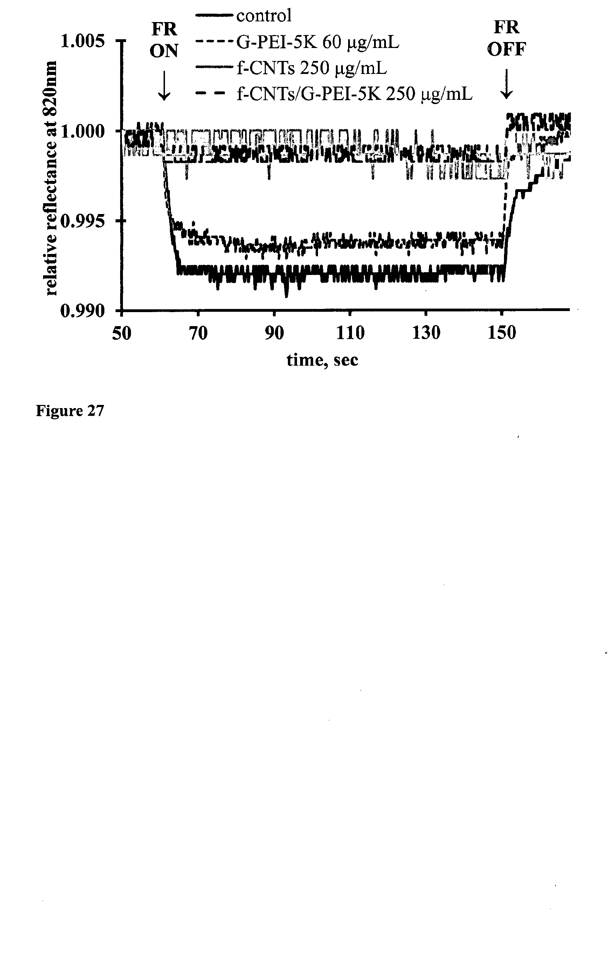

Further studies on the effect of the above mentioned materials on PSI reaction center, in terms of far-red photo-oxidable $P_{700+}$, in permeaplasts and spinach thylakoid membranes were performed. Figure 27 shows the effect of antibacterial compounds on absorbance/ reflectance curves at 820 nm in cyanobacterial permeaplasts. PSI activity measured as far-red induced absorbance/ reflectance changes at 820 nm, indicates the $P_{700}^+$ concentration. Figure 27 shows the relative absorption decrease for permeaplasts (solid line .. ) and for permeaplasts in the presence of G-PEI-5K (dashed line ˙ ˙ ! ''). The relative absorption decrease in the presence of f-CNTs/G-PEI-5K (dashed line .:'), and of f-CNTs, (solid tine ˙ ˙ |│ ) is slight, indicating an increase in PSI inhibition.

Tables 3, 4, and 5 show the effect of G-PEI-5K, f-CNTs/G-PEI-5K and f-CNTs, on permeaplast PSI activity respectively. The results reveal that f-CNTs/G-PEI-5K inhibits PS I activity up to ~89% (Table 4), while the corresponding value for f-CNTs is ~83% (Table 5). It is evident that f-CNTs/G-PEI-5K is the most effective compound compared to f-CNTs and G-PEI-5K respectively, being effective at lower concentrations.

**Studies on Photo- oxidative state of $P_{700}$ in spinach thylakoid membranes.**

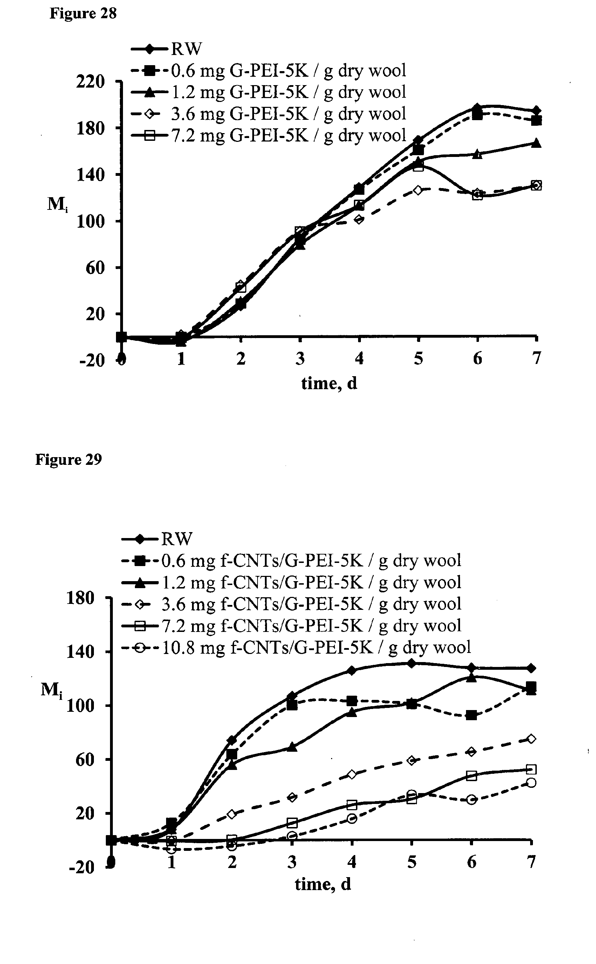

Figure 27 shows the effect of antibacterial compounds on absorbance/ reflectance curves at 820 nm in spinach thylakoid membranes. PSI activity is measured as far-red induced absorbance/ reflectance changes at 820 nm, indicating $P_{700}^+$ concentration. Figure 28 shows the relative absorption decrease for spinach thylakoid membranes (solid line ) and for spinach thylakoid membranes in the presence of G-PEI-5K (dashed line ). The relative absorption decrease in the presence of f-CNTs, (solid line ) is minor, indicating a slight inhibition in PSI. On the other hand, in the case of f-CNTs/G-PEI-5K (dashed line ), there is no change of the relative absorption, implying the total inhibition of PS I activity.

Tables 6, 7, and 8 show the effect of G-PEI-5K, f-CNTs/G-PEI-5K and f-CNTs, on thylakoid membrane PSI activity, respectively. It is remarkable that f-CNTs/G-PEI-5K inhibits almost totally (~99%) the thylakoid membrane PSI activity. The thylakoid membrane PSI activity is inhibited by ~84% in the presence of f-CNTs and ~19% in the presence of G-PEI-5K.

In conclusion, the hybrid material f-CNTs/G-PEI-5K totally inhibits the thylakoid membrane PSI activity. Moreover f-CNTs inhibit thylakoid membrane and permeaplast PSI activity in the same manner, while G-PEI-5K enhances the inhibition in spinach thylakoid membranes compared to cyanobacterial permeaplasts PSI activity.

**6. Textile materials with bacterial protection.**

**a. Coating of textiles with antibacterial hybrid materials (Antibacterial finishing of textiles).**

Commercial, untreated, 100% wool fabric (weight, 155 g·m$^{-2}$), with plain weave was used for the antibacterial finishing with G-PEI-5K, f-CNTs and f-CNTs/G-PEI-5K. The wool fabric was washed in a bath containing non-ionic washing agent (Levantin LNB). Dry wool fabrics, with known dry mass, were placed in 200 mL Erlenmeyer flasks with aqueous solutions of increasing concentrations of antibacterial materials, at a liquor-to-fabric ratio 100:1. The solutions were placed in an orbital shaker (Julabo SW22) under agitation at 120 rpm, at 25 °C, for 24 h. The concentrations of G-PEI-5K, f-CNTs and f-CNTs/G-PEI-5K in solutions were 0.6, 1.2, 3.6, 7.2 and 10.8 mg of substance per gram of dry wool mass.

**b. Antibacterial resistance after textile's washing.**

Three washing procedures were used to evaluate the durability of the antibacterial finishing. The wool samples, which were dipped in solutions with maximum concentrations of antibacterial substances, subjected to washing treatments with: i) standard detergent at 40 °C for 30 min (ISO 105-C10:2006) [vlvii], ii) with tetrachlorethylene (ISO 105-D01:1993) [xlviii] (dry cleansing procedure) and iii) with liquid $CO_2$. According to the first and second procedure, samples were washed in a Rotawash M228-SDL International machine. The washings were repeated 5 times. For the third method, samples were soaked in liquid $CO_2$ at room temperature and then were kept under constant $CO_2$ flow of 0.8 mL/min for 25 h. Liquid $CO_2$ washing was equivalent to fifty washings of 30 min each.

**c. Measurement of Antibacterial properties of textiles with hybrid materials**

Antibacterial properties of textile containing hybrid materials developed in this invention were assessed with a method proposed for the first time in the present invention, which correlates the increase of Chl a fluorescence to cell proliferation. The method was developed using *Synechococcus* sp. PCC7942 cells adapted on textiles treated using G-PEI-5K, f-CNTs και G-PEI-5K/f-CNTs as antibacterial agents, either on un-washed, as control, or washed as described previously.

A suitable quantity of cyanobacterial cells was harvested from the culture suspensions by centrifugation (5000 rpm, 5 min) and was re-suspended in buffered BG11, so that the concentration of Chi $\alpha$ was 52.0 $\mu$ g·mL$^{-1}$. Untreated and treated as above wool fabric samples were used as test specimens for the evaluation of the antibacterial activity. A drop of the abovementioned solution was pipetted (0.05 mL) on each test specimen producing a stain of less than 3.0 mm diameter on the fabric. These samples were dark-adapted for 15 min in a suitable clip and Chl $\alpha$ fluorescence was measured using a PEA-fluorometer (PEA, Hansatech Instruments Ltd, Norfolk, UK). Upon excitation of a dark-adapted photosynthetic sample, Chl $\alpha$ fluorescence decays from a higher to a lower steady level within ns. This transiently steady fluorescence, the first recorded signal lasting a few $\mu$s, is labeled as O while its intensity is usually denoted as F$_0$. Often, the F$_0$ is also designated by the equivalent terms constant fluorescence, initial fluorescence, or dark-level fluorescence. The measuring area is circular with a diameter of 3.0 mm and the measurements were made at 25 °C. The actinic light (peak at 650 nm) is supplied by an array of three light emitting diodes and is focused on the sample surface to provide a homogeneous irradiation of the exposed area. The PEA fluorometer provides continuous excitation at 650 nm (3000 $\mu$E·m$^{-2}$·s$_{-1}$; $\Delta_\lambda$ = 22 nm). It detects fluorescence at wavelengths above 700 nm (50% transmission at 720 nm) and records it continuously from 0.01 ms to 30 s with data acquisition every 0.01 ms for the first 0.3 ms, every 0.1ms between 0.3ms and 3 ms, every 1 ms between 3 ms and 30 ms, every 10 ms between 30 ms and 300ms, every 100 ms between 300 ms and 3000 ms and every 1000 ms thereafter.

In this example, raw fluorescence data collected during the first 10 s of illumination were used. The first reliable fluorescence value in the transient (at 50 $\mu$s) is used as the initial fluorescence value F$_0$, for the samples that had been kept in darkness for 15 min.

Cyanobacterial growth on wool and growth inhibition on antibacterial samples was monitored by measurement of the Chl $\alpha$ fluorescence every 24 h for seven days. Throughout the course of seven days the samples were kept in an incubator under white fluorescent light (100 $\mu$E·m$^{-2}$·s$^{-1}$) at 31 °C.

The % change (M$_i$) in Chi $\alpha$ fluorescence value $F_0$ of cyanobacteria after I days of incubation is calculated using the equation (1):

$$\text{Equation (1)}$$

$$M_i = \frac{F_{0_i} - F_{0_{c_{..}}}}{F_{0_n}} \times 100$$

Where:

$F_{o0}$ is the value of Chl $\alpha$ fluorescence of cyanobacteria at zero contact time and

$F_{o0}$ is the Chl $\alpha$ fluorescence value of cyanobacteria after 1, 2, ..., i days.

The material's antibacterial action is represented by the Bacterial Protection Index (BPI) $\Pi_7$, given by the equation (2):

$$\text{Equation (2)}$$

$$\Pi_7 = \frac{M_{U_I} - M_{T_7}}{M_{U_7}} \times 100$$

Where:

M$_{u7}$, is the change in the cyanobacterial Chl $\alpha$ F$_0$ value on the untreated sample after 7 days of incubation and
$M_{I7}$, is the change in the cyanobacterial Chl $\alpha$ F$_0$ value on the treated sample after 7 days of incubation.

**Evaluation of antibacterial properties of wool containing G-PEI-5K, f-CNTs, f-CNTs/G-PEI-5K by use of *Synechococcus* sp. PCC7942 cyanobacyeria**

The antibacterial properties of wool containing f-CNTs were not determined as poor adhesion of f-CNTs on wool and aggregate formation in the aqueous solution, prevented their homogenous dispersion on the wool surface.

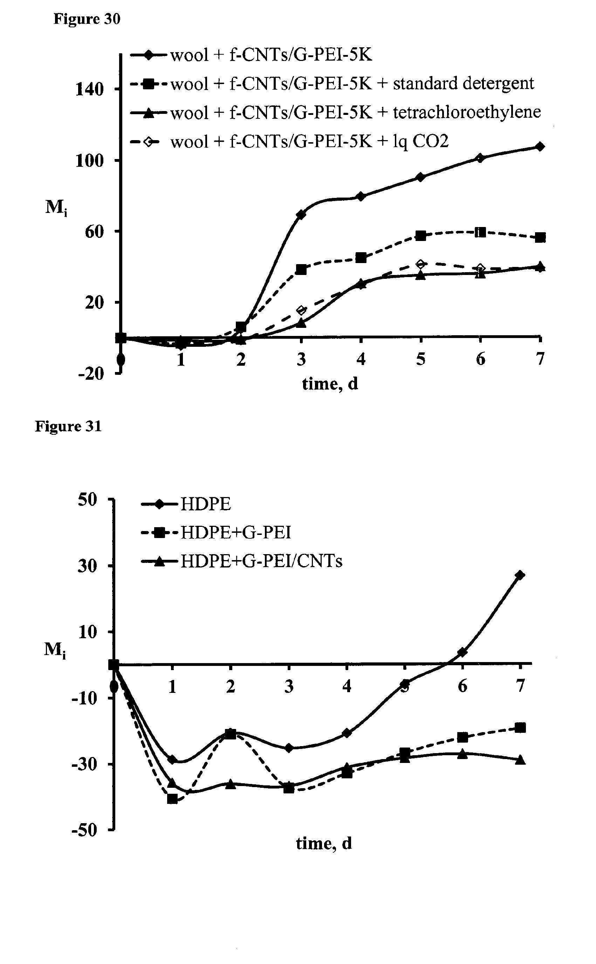

Figures 29 and 30 show cyanobacterial Chl *a* fluorescence ($F_0$) changes on wool/G-PEI-5K and wool/f-CNTs/G-PEI-5K adapted cells, respectively.

In order to validate the method it is necessary to confirm that the measured quantity $F_0$ is directly related to the bacterial population and that the test organism is sensitive to gram(-) antibacterials. To this end, daily cyanobacterial Chl a monitoring was performed on untreated wool samples with: 1) control cyanobacterial culture, 2) cyanobacterial culture after the addition of 20 $\mu$M DCMU [xlix], 3) Chl a extracted from cyanobacteria [39].

In control experiments of bacterial growth on wool, DCMU was used as a PSII inhibitor and showed that Chl $\alpha$ fluorescence ($F_0$) *a* of cuanobacteria remains stable against time. The same result was obtained with pure Chl a extract on wool. This indicated that increase in $F_0$ values can only be attributed to active cyanobacteria and not to any other form of Chl a coming from either dead or inactive cells.

In tables 9 and 10 the values of the Bacterial Protection Index $\Pi_7$ are presented for wool/G-PEI-5K and wool/f-CNTs/G-PEI-5K after seven days of incubation.

The wool/G-PEI-5K does not exhibit any significant antibacterial behaviour, while the antibacterial properties of wool/f-CNTs/G-PEI-5K are extremely high.

Samples containing 10.8 mg f-CNTs/G-PEI-5K per gr dry weight of wool were washed with a standard dry cleaning reagent (tetrachloroethylene) or with Liquid $CO_2$.

Figure 31 represents the $F_0$ difference of cyanobacterial Chl *a* on wool samples containing f-CNTs/G-PEI-5K washed either with tetraachloroethylene or with liquid $CO_2$.

In Table 11 the values of $\Pi_7$ of the wool/f-CNTs/G-PEI-5K after washing are presented. A decrease of $\Pi_7$ is observed for the sample washed with dry-cleaning detergent (5 cycles), while no significant changes are observed after liquid $CO_2$ washing (50 cycles), indicating very good adhesion of f-CNTs/G-PEI-5K onto wool surface resulting in a hybrid textile material which retains its antibacterial action after washing.

### 7. Thermoplastics with antibacterial properties.

### a. Incorporation of antibacterial hybrid material in thermoplastics

Commercial high density polyethylene (HDPE) was used for the preparation of antibacterial surfaces. G-PEI-5K, and f-CNTs/G-PEI-5K were incorporated in HDPE by an extrusion process using a single screw Johnson plastics extruder in an analogous process of pure HDPE [50]. Two HDPE composites contained 1.0 % w/w G-PEI-5K (HDPE/G-PEI-5K) and 1.0 % w/w f-CNTs/G-PEI-5K (HDPE/G-PEI-5K/f-CNTs).

### b. Antibacterial activity

The antibacterial properties of the hybrid composites were evaluated using the method described in this invention. The material's surfaces were disinfected with 70% (v/v) ethanol for 5 min, washed with hot water containing anionic detergent and finally rinsed with distilled water. Before the experiment, the surfaces were sprayed with 70% (v/v) ethanol and allowed to evaporate before bacterial loading.

Suitable quantity of cyanobacteria cells was harvested from the culture suspensions by centrifugation (5000 rpm, 5 min) and was resuspended in buffered BG11, so that the concentration of Chl $\alpha$ was 100.0 $\mu$g•mL$^{-1}$. A drop of the abovementioned solution was pipetted (0.025 mL) either on the surface of each test specimen producing a stain of less than 3.0 mm diameter or in a 0.4 cm diameter and 0.5 cm depth hole on each material.

### c. Evaluation of antibacterial properties of hybrid thermoplastics

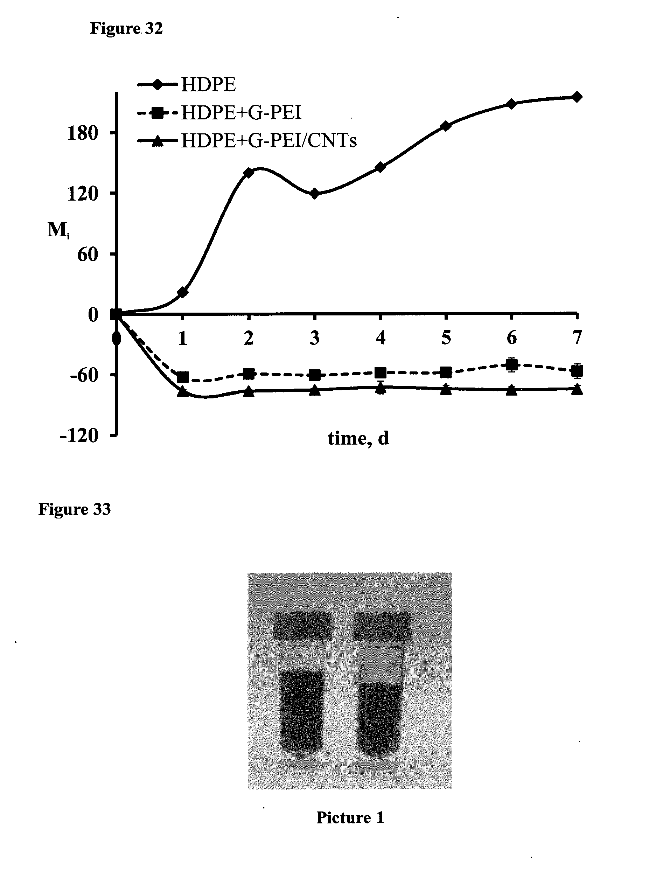

The results in Figure 32 represent the experimental data conducted on material's surfaces while Figure 33 shows the corresponding results for the experiments performed in holes.

The initial inhibition of growth of bacteria on their surface shown in Figure 32 can be attributed to the time needed for the cyanobacteria to adapt to the new conditions and create the appropriate substrate for growth. On the other hand this trend was not observed for the experiments carried out in holes (bulk) of the material (Figure 33) because the surface was roughened and most favored to the adhesion of bacteria. Regarding pure HDPE, the amount of cyanobacteria on surface increased by 27 % the seventh day (Figure 32). However, when the testing microorganisms were placed in the material's holes, cyanobacteria growth was not prevented at all, showing no antibacterial behavior at all. The corresponding increase of the amount of cyanobacteria was 213% at the end the seventh day. On the other hand, both HDPE composites exhibited remarkable bacteriocidal properties in all tested conditions. Specifically, the HDPE/G-PEI-5K composite reduced the amount of cyanobacteria by 19 %, when the bacteria applied on the surface, while the reduction reached the value of 58 % in the case of holes (Figure 33). Furthermore, the HDPE/G-PEI-5K/f-CNTs nanocomposite showed improved antibacterial properties compared to HDPE/G-PEI-5K composite. In this case, the reduction in the amount of microorganisms was 29 %, when the experiment performed on surface while the corresponding value reached 75 % for the experiment carried out in material's holes.

**Table 1.** Effect of G-PEI-5K, f-CNTs/G-PEI-5K and f-CNTs on cyanobacterial PS II activity (H$_2$O→PBQ, R; $\mu$mol O$_2$/mg Chl•h).

| | BG11 | G-PEI-5K 20μg/ml | G-PEI-5K 40μg/ml | G-PEI-5K 60μg/ml | f-CNTs 25μg/ml | f-CNTs 100μg/ml | f-CNTs 250μg/ml | f-CNTs/G-PEI-5K 25μg/ml | f-CNTs/G-PEI-5K 100μg/ml | f-CNTs/G-PEI-5K 250μg/ml |
|---|---|---|---|---|---|---|---|---|---|---|
| **R** | 119.88 | 107.13 | 71.42 | 50.16 | 119.03 | 98.63 | 74.82 | 44.02 | 37.88 | 35.83 |
| **R%** | **100.00** | **89.36** | **59.57** | **41.84** | **99.29** | **82.27** | **62.41** | **36.72** | **31.60** | **29.89** |

**Table 2.** Effect of G-PEI-5K, f-CNTs/G-PEI-5K and f-CNTs on cyanobacterial PS I activity (H$_2$O→PBQ, R; $\mu$mol O$_2$ / mg Chl•h)

| | BG11 | G-PEI-5K 20$\mu$g/ml | G-PEI-5K 40$\mu$g/ml | G-PEI-5K 60$\mu$g/ml | f-CNTs 25$\mu$g/ml | f-CNTs 100$\mu$g/ml | f-CNTs 250$\mu$g/ml | f-CNTs/G-PEI-5K 25$\mu$g/ml | f-CNTs/G-PEI-5K 100$\mu$g/ml | f-CNTs/G-PEI-5K 250$\mu$g/ml |
|---|---|---|---|---|---|---|---|---|---|---|
| **R** | 317.03 | 280.92 | 208.68 | 120.39 | 152.18 | 88.76 | 13.68 | 125.94 | 16.79 | 8.40 |
| **R%** | 100.00 | 88.61 | 65.82 | 37.97 | 48.00 | 28.00 | 4.31 | 39.72 | 5.30 | 2.65 |

**Table 3.** Effect of G-PEI-5K on permeaplast PSI activity.

| | $P700^+$ ($\triangle A_{820}/A_{820}$)(x$10^2$) | % αναστολή |
|---|---|---|
| **BG11** | 4.23 | |
| **G-PEI-5K 20μg/ml** | 4.05 | 4.29 |
| **G-PEI-5K 40μg/ml** | 3.88 | 8.21 |
| **G-PEI-5K 60μg/ml** | 3.79 | 10.30 |

**Table 4.** Effect of f-CNTs/G-PEI-5K on permeaplast PSI activity.

| | $P700^+$ ($\triangle A820/A820$)(x$10^2$) | % αναστολή |
|---|---|---|
| **BG11** | 4.23 | |
| **f-CNTs/G-PEI-5K 25μg/ml** | 3.05 | 27.98 |
| **f-CNTs/G-PEI-5K 50μg/ml** | 0.57 | 86.51 |
| **f-CNTs/G-PEI-5K 100μg/ml** | 0.68 | 83.95 |
| **f-CNTs/G-PEI-5K 250μg/ml** | 0.46 | 89.19 |

**Table 5.** Effect of f-CNTs on permeaplast PSI activity.

| | $P700^+$ ($\triangle A820/A820$)(x$10^2$) | % αναστολή |
|---|---|---|
| **BG11** | 4.23 | |
| **f-CNTs 50μg/ml** | 2.7 | 48.57 |
| **f-CNTs 100μg/ml** | 0.61 | 85.57 |
| **f-CNTs 250μg/ml** | 0.71 | 83.22 |

**Table 6.** Effect of G-PEI-5K, on spinach thylakoid membranes PSI activity.

| | $P^+_{700}$ ($\triangle A_{820}/A_{820}$)(x$10^2$) | % αναστολή |
|---|---|---|
| Ρυθμιστικό διάλυμα pH 6.5 | 3.22 | |
| **G-PEI-5K 20μg/ml** | 3.01 | 6.59 |
| **G-PEI-5K 40μg/ml** | 2.82 | 12.55 |
| **G-PEI-5K 60μg/ml** | 2.61 | 18.83 |

**Table 7.** Effect of f-CNTs on permeaplast PSI activity.

| | $P^{+700}$ $(\triangle A_{820}/A_{820})(x10^2)$ | % αναστολή |
|---|---|---|
| Ρυθμιστικό διάλυμα pH 6.5 | 3.22 | |
| f-CNTs/G-PEI-5K | 1.64 | 49.00 |
| f-CNTs/G-PEI-5K | 4.45 | 55.05 |
| f-CNTs/G-PEI-5K | 0.78 | 76.40 |
| f-CNTs/G-PEI-5K | 0.64 | 80.12 |
| f-CNTs/G-PEI-5K | 0.28 | 91.29 |
| f-CNTs/G-PEI-5K | 0.04 | 98.87 |

**Table 8.** Effect of f-CNTs on spinach thylakoid membranes PSI activity.

| | $P^{+700}$ $(\triangle A_{820}/A_{820})(x10^2)$ | % αναστολή |
|---|---|---|
| Ρυθμιστικό διάλυμα pH 6.5 | 3.22 | |
| f-CNTs 10μg/ml | 2.68 | 16.76 |
| f-CNTs 25μg/ml | 2.30 | 28.61 |
| f-CNTs 50μg/ml | 2.37 | 26.45 |
| f-CNTs 100μg/ml | 0.91 | 71.80 |
| f-CNTs 150μg/ml | 0.81 | 74.72 |
| f-CNTs 250μg/ml | 0.50 | 84.42 |

**Table 9.** Bacterial Protection Index $\Pi_7$ of G-PEI-5K on textile.

| | 0.6[1] | 1.2[1] | 3.6[1] | 7.2[1] |
|---|---|---|---|---|
| $\Pi_7$ | 4.3 | 14.1 | 33.0 | 33.1 |

[1]mg G-PEI-5K per gr dry wool.

**Table 10.** Bacterial Protection Index $\Pi_7$ of f-CNTs/G-PEI-5K on textile.

| | 0.6[1] | 1.2[1] | 3.6[1] | 7.2[1] | 10.8[1] |
|---|---|---|---|---|---|
| $\Pi_7$ | 10.7 | 12.6 | 41.2 | 59.1 | 66.9 |

[1]mg f-CNTs/G-PEI-5K per gr dry wool.

**Table 11.** Bacterial Protection Index $\Pi_7$ of f-CNTs/G-PEI-5K on textile (10.8 mg nanocomposite per gr dry wool) under several washing treatments

| | Detergent | Dry clean | Liquid $CO_2$ |
|---|---|---|---|
| $\Pi_7$ | 47.8 | 62.9 | 63.7 |

## References

i

_] . S. B. Liu, T. H. Zeng, M. Hofmann, E. Burcombe, J. Wei, R. Jiang, J. Kong, Y. Chen, , *ACS Nano*, **2011**, 5, 6971 6980.

ii

! . S. B. Liu, L. Wei, L. Hao, N. Fang, M. W. Chang, R. Xu, Y. Yang, Y. Chen, Sharper and Faster "Nano Darts" Kill More Bacteria: A Study of Antibacterial Activity of Individually Dispersed Pristine Single-Walled Carbon Nanotube, *ACS Nano*, **2009**, 3, 3891–3902.

iii

_] . S. Kang, M. S. Mauter, M. Elimelech, , *Environ. Sci. Technol.*, **2008**, 42, 7528 7534.

iv

| | . S. Kang, M. Pinault, L.D. Pfefferle, M. Elimelech, , *Langmuir*, 2007, 23, 8670–8673.

v

|‾ . W. Hu, C. Peng, W. Luo, M. Lv, X. Li, D. Li, Q. Huang, C. Fan, , *ACS Nano*, **2010**, 4, 4317–4323.

vi

_] . L. R. Arias, L. J. Yang, , *Langmuir*, **2009**, 25, 3003–3012.

vii

! . Y. Bai, H Song Park, S. J. Lee, T. S. Bae, F. Watari, M. Uo, M. H. Lee, , *Carbon*, **2011**, 49, 3663 3671.

viii

.! . R. Mohan, A. M. Shanmugharaj, R. Sung Hun, , *J. Biomed. Mat. Res. Part B: Appl. Biomat.*, **2011**, 96B, 119 126.

ix

. D. Pantarotto, C. D. Partidos, J. Hoebeke, F. Brown, E. Kramer, J.-P. Briand, S. Muller, M. Prato, A. Bianco, Immunization with Peptide-Functionalized Carbon Nanotubes Enhances Virus-Specific Neutralizing Antibody Responses, *Chemistry and Biology*, 2003, *10*, 961 966.

x . A. Niu, Y. Han, J. Wu, N. Yu, Q. Xu, Synthesis of One-Dimensional Carbon Nanomaterials Wrapped by Silver Nanoparticles and Their Antibacterial Behavior, *The Journal of Physical Chemistry C*, 2010, 114, 12728 12735.

xi . E. Murugan, G. Vimala, Effective functionalization of multiwalled carbon nanotube with amphiphilic poly(propyleneimine) dendrimer carrying silver nanoparticles for better dispersability and antimicrobial activity, *Journal of Colloid and Interface Science*, 2011, *357*, 354–365.

xii . J. Zhou, X. Qi, Multi-walled carbon nanotubes epilson polylysine nanocomposite with enhanced antibacterial activity, *Letters in Applied Microbiology*, 2011, 52, 76–83

xiii. L. Yan, F. Zhao, S. Li, Z. Hu, Y. Zhao, HYPERLINK "http://pubs.rsc.org/en/content/articlelanding/2011/nr/c0nr00647e"Low-toxic and safe nanomaterials by surface-chemical design, carbon nanotubes, fullerenes, metallofullerenes, and graphenes, *Nanoscale*, 2011, 3, 362 382.

xiv. T. S. Ryge, P. R. Hansen, Novel lysine-peptoid hybrids with antibacterial properties, *Journal of Peptide Science*, **2005**, *11*, 727 734.

xv . M. R. Yeaman, N. Y. Yount, Mechanisms of Antimicrobial Peptide Action and Resistance, *Pharmacological Reviews*, 2003, 55, 27–55.

xvi. X. P. Wang, H. Y. Han, X. Q. Liu, X. X. Gu, K. Chen, D. L. Lu, Multi-walled carbon nanotubes can enhance root elongation of wheat (Triticum aestivum) plants, *J. Nanopart. Res.*, 2012, *14*, 841–850.

xvii. M. V. Khodakovskaya, K. Silva, D. A. Nedosekin, E. Dervishic, A. S. Birisa, E. V. Shashkov, E. I. Galanzha, V. P. Zharov, *Proc. Natl. Acad. Sci.*, 2011, *108*, 1029 1033.

xviii. F. Schwab, T. D. Bucheli, L. P. Lukhele, A. Magrez, B. Nowack, L. Sigg and K. Knaucr, *Environ. Sci. Technol.*, 2011, *45(14)*, 6136–6144.

xix. P. Boldog, K. Hajdu, M. Magyar, É. Hideg, K. Hernádi, E. Horváth, A. Magrez, K. Nagy, G. Váró, L. Forró, L. Nagy, Carbon nanotubes quench singlet oxygen generated by photosynthetic reaction centers, *Physica Status Solidi (b)*, 2013, *250(12)*, 2539 2543.

xx. M. T. Madigan, J. M. Martinko, D. A. Stahl, D. P. Clark, Brock Biology of Microorganisms, 13th ed., *Benjamin Cummings*, 2012.

xxi. N. Nelson, C. F. Yocum, Structure and Function of Photosystems I and II, *Annu. Rev. Plant Biol.*, 2006, *57*, 521 565.

xxii. J. C. Streibig, F. E. Dayan, A. M. Rimando, S. O. Duke, *Pestic Sci*, 1999, *55*, 137–146.

xxiii. W. Tischek, H. Strotmann, *Biochimica et Biophysica Acta*, 1977, *460*, 113-125.

xxiv. D. Hofer, In U. C. Hipler, & P. Elsner, Antimicrobial textiles-Evaluation of their effectiveness and safety in biofunctional textiles and the skin, Basel-Karger, 2006, 42–50.

xxv. B. G. Joiner, Determining Antimicrobial Efficacy and Biocompatibility of Treated Articles using Standard Test Methods, In Bioactive Fibres and Polymers, J. V. Edwards, T. L. Vigo, *American Chemical Society*, Washington DC, 2001, 201–217.

xxvi. P.D. Askew, Measuring activity in antimicrobial textiles, *Chemistry Today*, 2009, *27*, 16 20.

xxvii. I. C. Gouveia, Nanobiotechnology: A new strategy to develop non-toxic antimicrobial Textiles, In A. Méndez-Vilas, Current Research, Technology and Education, *Topics in Applied Microbiology* and *Microbial Biotechnology*, 2010, Badajoz, 407-414.

xxviii. B. Tanner, Antimicrobial Fabrics – Issues and Opportunities in the Era of Antibiotic Resistance, *AATCC Review*, 2009, *9(11)*, 30-33.

xxix. M. M. G. Fouda, E. S. Abdel-Halim, S. S. Al-Deyab, Antibacterial modification of cotton using nanotechnology, *Carbohydrate Polymers*, 2013, *92*, 943 954.

xxx. Y. Gao, R. Cranston, Recent Advances in Antimicrobial Treatments of Textiles, *Textile Research Journal*, 2008, *78*, 60-72.

xxxi. H. W. Swofford, An Overview of Antimicrobial Testing for Textile Applications, *AATCC Review*, 2010, *10(6)*, 51-55.

xxxii. L.-A. Tziveleka, A.-M. G. Psarra, D. Tsiourvas, C. M. Paleos, Synthesis and characterization of guanidinylated poly(propylene imine) dendrimers as gene transfection agents, *Journal of Controlled Release*, 2007, *117*, 137–146.

xxxiii. Z. Sideratou, D. Tsiourvas, C. M. Paleos, Quaternized Poly(propylene imine) Dendrimers as Novel pH-Sensitive Controlled-Release Systems, *Langmuir*, 2000, *16*, 1766 1769.

xxxiv. R. Rippka, J. Deruelles, J. B. Waterbury, M. Herdman, R. T. Stanier, Generic assignments, strain histories and properties of pure cultures of cyanobacteria, *J. Gen. Microbiol.*, 1979, *111*, 1-61.

xxxv. R. Rippka, Isolation and purification of cyanobacteria, *Methods in Enzymology*, 1988, *167*, 3-27.

xxxvi. K. Stamatakis, G. C. Papageorgiou, The osmolality of the cell suspension regulates phycobilisome-to-photosystem I excitation transfers in Cyanobacteria, *Biochimica et Biophysica Acta*, 2001, *1506*, 172-181.

xxxvii. G. C. Papageorgiou, T. Lagoyanni, Photosynthetic properties of rapidly permeabilized cells of the cyanobacterium Anacystis nidulans, *Biochimica et Biophysica Acta*, 1985, *807*, 230-237.

xxxviii. G. C. Papageorgiou, Rapid permeabilization of Anacystis nidulans to electrolytes, *Meth. Enzymol*, 1988, *167*, 259-262.

xxxix. P. Moran, Formulae for determination of chlorophyllous pigments extracted with N,N-dimethyl-formamide, *Plant Physiol.*, 1982, *69*, 1376-1381.

xl. D. S. Gorman, R. P. Levine, Cytochrome f and plastocyanin: their sequence in the photosynthetic electron transport chain of Chlamydomonas reinhardii, *Proc. Natl. Acad. Sci.*, 1965, *54*, 1665-1669.

xli . M. Chrysina, G. Zahariou, Y. Sanakis, N. Ioannidis, V. Petrouleas, Conformational changes of the S2YZ. intermediate of the S2 to S3 transition in photosystem II, *Journal of Photochemistry and Photobiology B: Biology*, **2011**, *104*, 72–77.

xlii . A. Trebst, E. Pistorius, F. (1965) Photosynthetische Reaktionen in UV-betrahlten Chloroplasten, *Z. Naturforsch*, **1965**, *20B*, 885–889.

xliii . U. Schreiber, C. Klughammer, C. Neubauer, Measuring P700 absorbance changes around 830 nm with a new type of pulse modulation system, *Z. Naturforsch.*, **1988**, *43C*, 686–698.

xliv . C. Klughammer, U. Schreiber, Analysis of light-induced absorbancy changes in the near-infrared spectral region. 1. Characterization of various components in isolated chloroplasts, *Z. Naturforsch.*, **1991**, *46C*, 233–244.

xlv . A. G. Ivanov, R. M. Morgan, G. R. Gray, M. Y. Velitchkova, N. P. A. Huner, Temperature/light dependent development of selective resistance to photoinhibition of photosystem I, *FEBS Letters*, **1998**, *430*, 288-292.

xlvi . E. Wientjes, R. Croce, PMS: Photosystem I electron donor or fluorescence quencher, *Photosynth Res.*, **2012**, *111*,185–191.

xlvii . ISO 105-C10:2006, Textiles - Tests for colour fastness - Part C10: Colour fastness to washing with soap, *ISO*, **2006**.

xlviii . BS EN ISO 105-D01:1993, Textiles - Tests for colour fastness - Part D01: Colour fastness to dry cleaning (with tetrachloroethylene solvent), *BSI*, **1993**.

xlix

**Claims**

1. Hybrid nanocomposite materials which are obtained by the electrostatic interaction of dendritic polymers with symmetrical or asymmetrical architecture, i.e., dendrimers or hyperbranched polymers, functionalized with positively charged groups with nanostructured carbon-based materials containing negatively charged moieties.

2. Hybrid nanocomposite materials according to claim 1 where positively charged groups are guanidinium moieties or quaternary ammonium groups or both or any other positively charged moieties which can be attached at dendritic scaffold.

3. Hybrid nanocomposite materials according to claims 1 and 2, where the nanostructured carbon-based materials are oxidized carbon nanotubes (f-CNTs) or graphene oxide (GO) or oxidized carbon nanodisks (f-CNDs) or their combinations.

4. Hybrid nanocomposite materials according to any one of claims 1 to 3, where the minimum loading of functional dendritic polymer in hybrid nanocomposite materials is 5 % w/w.

5. Hybrid nanocomposite materials according to any one of claims 1 to 4 which present antibacterial properties at minimum concentration 5 $\mu$g·mL$^{-1}$.

6. Hybrid nanocomposite materials according to any one of claims 1 to 5 which present complete inhibition (up 98%) of the activity of photosystem I.

7. Hybrid nanocomposite materials according to any one of claims 1 to 6 which present inhibition at least 50% of the activity of photosystem II.

8. Hybrid nanocomposite materials according to any one of claims 1 to 7, which comprise functionalized dendritic polymers with symmetrical or asymmetrical architecture, derived from commercially available dendritic polymers such as:

   • Hyperbranched polyethylenimine with molecular weight 400-750.000 Da,
   • Diaminobutane propyleneimine dendrimers (DAB) with molecular weight 200-7.000 Da,
   • Poly(amido amine) (PAMAM) dendrimers with molecular weight 200-150.000 Da,
   • other dendritic polymers exhibiting similar characteristics

and are functionalized with positively charged groups at 10-100% molecular coverage.

**9.** Method for the preparation of hybrid nanocomposite materials according to any one of claims 1 to 8, which comprises the following steps:

- Dispersion in water or in any other solvent with the aid of ultrasound of one part of each nanostructured carbon-based material and subsequent addition 3 parts of functionalized dendritic polymers with symmetric or asymmetric architecture, dissolved in same solvent, bearing positively charged groups,
- Ultrasound of the resulting dispersions and then stirring for 12 hours,
- Ultracentrifugation and washing with appropriate solvent at least once, preferably three times,
- Lyophilization or evaporation of solvent to obtain the final hybrids in the form of powder.

**10.** Method for the preparation of textile material (woven and nonwoven), metal, wood, glass, plastics, thermoplastics, synthetic or other composite material with bacterial protection which is prepared by the incorporation or coating of the nanocomposite hybrid materials of any one of claims 1 to 9 to the said substrate.

**11.** Method according to claim 10 where the lower loading of hybrid nanocomposite materials according to any one of claims 1 to 9 in order to achieve fully bacterial protection is 1 % w/w.

**12.** Method for the quantitative determination of antibacterial properties of any compound (organic, inorganic, natural or man-made) incorporated to any material or substrate (polymers, plastics, thermoplastics, metal, wood, woven and nonwoven textile materials, etc.) which

- correlates the dark-level fluorescence value ($F_0$) of cyanobacteria chlorophyll $\alpha$ (Chl $\alpha$) at zero time after dark acclimation ($F_0$) to the increase or decrease of their population and
- quantitatively determines the degree of bacterial protection in conditions analogous to that of the intended use of each material or substrate.

**13.** Method according to claim 12, which comprises the following steps:

- incorporation of hybrid nanocomposite materials of claims 1 to 9 or any other material that exhibits antibacterial properties to the substrate / material (any),
- incubation of cyanobacteria directly on the material,
- measurement of $F_0$ on the material,
- determination of the growth of bacteria on the material based on the value of fluorescence measured at specified intervals and from the change of fluorescence value,
- increase of the measured fluorescence value is due to the growth of bacteria population leading to the conclusion that the material does not exhibit antibacterial properties, while if the fluorescence value is low, stable, or decreasing, the material exhibits antibacterial properties.

Figure 1

Figure 2

Figure 3

Figure 4

Figure 5

Figure 6

Figure 7

Figure 8

**Figure 9**

**Figure 10**

Figure 11

Figure 12

**Figure 13**

**Figure 14**

Figure 15

Figure 16

Figure 17

Figure 18

**Figure 19**

**Figure 20**

**Figure 21**

**Figure 22**

Figure 23

Figure 24

**Figure 25**

**Figure 26**

Figure 27

**Figure 28**

**Figure 29**

**Figure 30**

Legend:
- wool + f-CNTs/G-PEI-5K
- wool + f-CNTs/G-PEI-5K + standard detergent
- wool + f-CNTs/G-PEI-5K + tetrachloroethylene
- wool + f-CNTs/G-PEI-5K + lq CO2

$M_i$ vs time, d

**Figure 31**

Legend:
- HDPE
- HDPE+G-PEI
- HDPE+G-PEI/CNTs

$M_i$ vs time, d

**Figure 32**

**Figure 33**

Picture 1